(19) Europäisches Patentamt
European Patent Office
Office européen des brevets

(11) **EP 4 625 428 A1**

(12) **EUROPEAN PATENT APPLICATION**
published in accordance with Art. 153(4) EPC

(43) Date of publication:
01.10.2025 Bulletin 2025/40

(21) Application number: 23914617.8

(22) Date of filing: 30.12.2023

(51) International Patent Classification (IPC):
*G16H 15/00* (2018.01)    *A61B 5/00* (2006.01)
*G16H 20/30* (2018.01)    *G16H 20/60* (2018.01)
*G16H 50/30* (2018.01)

(52) Cooperative Patent Classification (CPC):
A61B 5/00; G16H 15/00; G16H 20/30; G16H 20/60;
G16H 50/30

(86) International application number:
PCT/CN2023/143705

(87) International publication number:
WO 2024/146486 (11.07.2024 Gazette 2024/28)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 03.01.2023  CN 202310003669
08.03.2023  CN 202310258733

(71) Applicant: Huawei Technologies Co., Ltd.
Shenzhen, Guangdong 518129 (CN)

(72) Inventors:
• ZHANG, Jie
  Shenzhen, Guangdong 518129 (CN)
• WANG, Nian
  Shenzhen, Guangdong 518129 (CN)
• SUN, Yuning
  Shenzhen, Guangdong 518129 (CN)
• XIE, Songlin
  Shenzhen, Guangdong 518129 (CN)

(74) Representative: Pfenning, Meinig & Partner mbB
Patent- und Rechtsanwälte
Theresienhöhe 11a
80339 München (DE)

(54) **BLOOD GLUCOSE MANAGEMENT METHOD AND RELATED ELECTRONIC DEVICE**

(57)    This application discloses a blood glucose management method and a related electronic device. In the method, meal time of a diet event of a user can be determined based on a blood glucose value of the user, and when a blood glucose curve drawn based on blood glucose values of the user is displayed, the meal time and/or a blood glucose value related to the meal time are/is displayed on the blood glucose curve. In this way, the diet event of the user can be associated with blood glucose of the user, and a blood glucose change of the user before and after a meal can be highlighted. This helps the user more intuitively learn of impact of a diet on the blood glucose, and helps the user manage and control the blood glucose starting from the diet.

FIG. 2

## Description

This application claims priorities to Chinese Patent Application No. 202310003669.5, filed with the China National Intellectual Property Administration on January 3, 2023 and entitled "BLOOD GLUCOSE DETECTION METHOD AND ELECTRONIC DEVICE", and to Chinese Patent Application No. 202310258733.4, filed with the China National Intellectual Property Administration on March 8, 2023 and entitled "BLOOD GLUCOSE MANAGEMENT METHOD AND RELATED ELECTRONIC DEVICE", both of which are incorporated herein by reference in their entireties.

## TECHNICAL FIELD

This application relates to the field of terminal technologies, and in particular, to a blood glucose management method and a related electronic device.

## BACKGROUND

Diabetes is a group of metabolic diseases characterized by hyperglycemia and caused by insulin secretion defects and biological dysfunctions. Diabetes is a long-term chronic disease. Daily behavior and self-management capabilities of users are one of the key factors that affect a diabetes control status. Therefore, control over diabetes requires systematic self-management of the users.

## SUMMARY

This application provides a blood glucose management method and a related electronic device, to associate an exogenous event with blood glucose of a user by using a blood glucose value of the user, help the user manage and control impact of the exogenous event on the blood glucose, and implement more effective self-management of the user on the blood glucose of the user.

According to a first aspect, an embodiment of this application provides a blood glucose management method. The method is applied to an electronic device, and the method includes: first obtaining a fasting blood glucose value; if blood glucose values of a user within a first time period exceed the fasting blood glucose value by a first threshold, determining first meal time based on the blood glucose values within the first time period; and displaying the first meal time and/or a blood glucose value related to the first meal time on a displayed first blood glucose curve after the first meal time is determined, where the first blood glucose curve indicates a blood glucose change of the user within the first time period.

According to the method provided in this embodiment of this application, the user may learn of a blood glucose change trend through an interaction interface of the electronic device. In addition, meal time is displayed, so that the user can more intuitively and clearly learn of an association between a diet and blood glucose, and the user can obtain impact of a diet event on the blood glucose. In this way, the user can better manage and control a blood glucose concentration change, to implement self-management of the user on the blood glucose of the user. In addition, for a diabetic patient, a diabetes self-management user can have a more positive attitude, richer diabetes knowledge, and better diabetes self-management behavior, and have more confidence in overcoming the disease.

With reference to the first aspect, in an implementation, the blood glucose value related to the first meal time may include one or more of the following: a blood glucose value at the first meal time, and a blood glucose value at a time point first duration before or after the first meal time.

Blood glucose values at key time points near the meal time are highlighted, so that the user can more clearly learn of a blood glucose status before and after a meal.

With reference to the first aspect, in an implementation, the first meal time is between a first time point and a second time point. The first time point is a time point of a blood glucose peak within the first time period, the second time point is before the first time point, and duration between the second time point and the first time point is second duration. The blood glucose value at the first meal time is greater than the fasting blood glucose value, and between the first time point and the second time point, the first meal time may be a $1^{st}$ time point at which a blood glucose rising rate of the user is greater than a second threshold.

The meal time is calculated based on the blood glucose and a relationship between the blood glucose and the diet, so that the meal time of the diet event of the user can be accurately calculated. In this way, the meal time can be accurately calculated based on a blood glucose value of the user without a manual input of the user. This facilitates an operation of the user, and also avoids a case in which an incorrect input may occur when the user performs the manual input.

With reference to the first aspect, in an implementation, the fasting blood glucose value may be obtained in the following three manners:

(1) The fasting blood glucose value is determined based on blood glucose values of the user within a second time period, and the fasting blood glucose value is a lower quartile of the blood glucose values within the second time period.

(2) The fasting blood glucose value is a lower quartile of blood glucose values between two meals of the user within the second time period.

(3) The fasting blood glucose value is determined based on second meal time, the second meal time is before the first meal time, and the fasting blood glucose value is an average blood glucose value within third duration before the second meal time.

It can be learned that the fasting blood glucose value of the user can be accurately calculated based on the blood glucose value of the user and the diet event of the user.

With reference to the first aspect, in an implementation, the electronic device may display a second blood glucose curve and/or a third blood glucose curve. The second blood glucose curve indicates a fasting blood glucose value on each of N days, and the third blood glucose curve indicates a blood glucose value related to the first meal time on each of the N days, where N≥1 and N is an integer.

The interaction interface of the electronic device displays a blood glucose change of the user within a period of time, so that the user can view, from a long-term perspective, the blood glucose change within the period of time and an association between the diet event and the blood glucose change, and the user can make an overall dietary adjustment to cope with an abnormal blood glucose change.

With reference to the first aspect, in an implementation, the method further includes: modifying the first meal time based on a user operation; and then displaying, on the first blood glucose curve displayed by the electronic device, modified first meal time and/or a blood glucose value related to the modified first meal time.

In other words, the user may manually modify the meal time, so that the electronic device can display more accurate information.

With reference to the first aspect, in an implementation, the method further includes: The electronic device may display calories of food and/or a proportion of each nutrient.

In this way, in addition to viewing the blood glucose change and the meal time of the user, the user can further learn of related information of the food consumed by the user during the meal. This helps the user more comprehensively learn of a current meal status.

According to a second aspect, an embodiment of this application further provides a blood glucose management method. The method is applied to an electronic device, and the method includes: first determining a meal volume and a meal speed of a user during a meal; obtaining an actual blood glucose curve of the user, where the actual blood glucose curve may indicate a blood glucose change of the user during the meal; obtaining a standard blood glucose curve of healthy people, where the standard blood glucose curve indicates a blood glucose change of the healthy people during a meal with the meal volume and the meal speed; and finally evaluating diet health of the user based on the actual blood glucose curve of the user and the standard blood glucose curve of the healthy people, where a larger difference between the actual blood glucose curve and the standard blood glucose curve indicates an unhealthier diet of the user, and a smaller difference between the actual blood glucose curve and the standard blood glucose curve indicates a healthier diet of the user.

According to the method provided in this embodiment of this application, a difference between blood glucose of the user and normal blood glucose of the healthy people can be quantified based on a meal status of the user, to help the user evaluate, based on blood glucose values during the meal, whether a blood glucose response of the user to a diet is healthy, help the user more clearly learn of a physical condition of the user, and facilitate self-management on blood glucose of the user.

With reference to the second aspect, in an implementation, obtaining the standard blood glucose curve of the healthy people specifically includes: obtaining blood glucose curves of a plurality of members in the healthy people, where the blood glucose curve indicates a blood glucose change of the member during the meal with the meal volume and the meal speed. In this way, the standard blood glucose curve of the healthy people can be determined based on the blood glucose curves of the plurality of members.

It can be learned that, in the method, the standard blood glucose curve of the healthy people can be calculated based on the blood glucose curves of the plurality of members with healthy blood glucose, so that the standard blood glucose curve can be more representative and more accurate when representing a blood glucose status of the healthy people.

With reference to the second aspect, in an implementation, determining the standard blood glucose curve of the healthy people based on the blood glucose curves of the plurality of members may include the following steps: (1) performing stretching or compression transformation on the blood glucose curves of the plurality of members in time domain, and/or performing stretching or compression transformation on the blood glucose curves of the plurality of members in amplitude, so that average values of the blood glucose curves of the plurality of members in time domain and amplitude are equal; and (2) performing point-by-point average value calculation on transformed blood glucose curves of the plurality of members, and determining a curve formed by connecting the average values as the standard blood glucose curve.

It can be learned that the standard blood glucose curve is obtained by normalizing the blood glucose curves of the plurality of members, and the standard blood glucose curve integrates blood glucose changes of the plurality of members.

With reference to the second aspect, in an implementation, the method further includes: obtaining meal-related data of the user during the meal, where the meal-related data includes heart rate data, exercise data, and emotion and stress data. Determining the meal volume and the meal speed of the user during the meal specifically includes: inputting the meal-related data of the user during the meal into a meal model, to obtain, through identification, the meal volume and the meal speed of the user during the meal, where the meal model is obtained through training based on meal-related data during a meal with a known meal volume and meal speed.

In this way, the user does not need to collect statistics on or record the meal volume or the meal speed, and the electronic device may obtain, through calculation, an accurate meal volume and meal speed of the user by obtaining data during the meal. This facilitates an operation of the user.

With reference to the second aspect, in an implementation, before obtaining the meal-related data of the user during the meal, the method further includes: detecting that the user confirms an operation of starting the meal, or detecting that data collected by a sensor satisfies a preset condition.

In other words, this embodiment of this application provides two methods for identifying that the user starts the meal: (1) identifying based on the user operation; and (2) identifying based on the data collected by the sensor. In this way, the meal-related data of the user during the meal may be obtained after it is identified that the user starts the meal, to calculate the accurate meal volume and meal speed.

With reference to the second aspect, in an implementation, the data collected by the sensor may indicate a heart rate, exercise, and emotion and stress of the user, and the preset condition includes that a difference between the heart rate of the user and a resting heart rate is still greater than a third threshold after impact of the exercise, and the emotion and stress is excluded.

It can be learned that, in the method, a heart rate change of the user during the meal and the impact of the exercise, and the emotion and stress of the user on the heart rate of the user are cleverly used, and the data that is collected by the sensor and that is used to reflect the heart rate, the exercise, and the emotion and stress of the user is used to identify a time point at which the user starts the meal, to achieve an accurate meal identification purpose.

With reference to the second aspect, in an implementation, evaluating the diet health of the user by using the actual blood glucose curve and the standard blood glucose curve specifically includes: separately extracting features of the actual blood glucose curve and the standard blood glucose curve, where the features may include one or more of the following: a maximum value of a single blood glucose change, non-stationary blood glucose time, quantities of blood glucose values in different blood glucose change rate categories, a quantity of blood glucose fluctuation times, time and amplitude of the single blood glucose change, and a curve envelope; and then evaluating the diet health of the user based on a difference between one or more features of the actual blood glucose curve and the one or more features of the standard blood glucose curve. A larger difference indicates an unhealthier diet of the user, and a smaller difference indicates a healthier diet of the user.

In other words, the features of the curves may be extracted to compare a difference between the two curves and quantify the difference between the curves, to help the user clearly learn of a difference between a blood glucose curve of the user and a blood glucose curve of the healthy people.

With reference to the second aspect, in an implementation, the method further includes: calculating an overall health score of the user based on a difference during each of N meals of the user within a third time period, where a larger overall health score indicates an unhealthier diet of the user within the third time period, and a smaller overall health score indicates a healthier diet of the user within the third time period.

It can be learned that, in the method, a health status of a plurality of diets of the user within a period of time can be evaluated, to help the user better learn of a physical condition of the user within the period of time.

With reference to the second aspect, in an implementation, the method further includes: The electronic device may display the actual blood glucose curve and the standard blood glucose curve.

In this way, the user can directly view a blood glucose status of the user and a blood glucose difference between the user and the healthy people through the interaction interface of the electronic device.

According to a third aspect, an embodiment of this application further provides a blood glucose management method. The method is applied to an electronic device, and the method includes: obtaining first exercise reference data of a user before exercise; outputting exercise prompt information based on the first exercise reference data, where the exercise prompt information indicates a recommendation for the exercise of the user; obtaining second exercise reference data of the user during the exercise; outputting an exercise alarm if the second exercise reference data reflects that an exercise risk of the user exceeds a fourth threshold; obtaining third exercise reference data of the user after the exercise ends; and outputting an exercise evaluation report of the exercise of the user based on the third exercise reference data, where the exercise evaluation report is used to evaluate impact of the exercise on the user. The first exercise reference data, the second exercise reference data, and the third exercise reference data include blood glucose and a parameter used to reflect a vital sign of the user.

According to the method provided in this embodiment of this application, the electronic device can monitor a physical condition of the user in an entire process from a moment before the user exercises to a moment after the user exercises, output the recommendation for the exercise when the user exercises, output the exercise alarm during the exercise of the user, and evaluate the exercise of the user after the exercise of the user ends, to avoid as much as possible that excessively high or low blood glucose harms the body of the user during the exercise, and help the user exercise in a healthier and safer way.

With reference to the third aspect, in an implementation, before obtaining the first exercise reference data of the user before the exercise, the method further includes: detecting an operation entered by the user for indicating that the exercise is to start, or detecting that current time reaches exercise time preset by the user.

It can be learned that, after identifying that the user is to start the exercise, the electronic device may start to obtain the first exercise reference data. Two methods for identifying that the user is to start the exercise are provided: (1) The user actively enters to start the exercise. (2) The current time reaches the exercise time preset by the user. In this way, before the user starts the exercise, a current exercise risk of the user can be evaluated in time based on the physical condition of the user, and the user is prevented in time from exercising when the exercise risk is high, so that the user can exercise when the exercise risk is low.

With reference to the third aspect, in an implementation, outputting the exercise prompt information based on the first exercise reference data specifically includes: performing weighted summation on levels of all data in the first exercise reference data to obtain a first exercise risk score through calculation, where a larger deviation of a data value of data from a normal range indicates a higher level of the data.

When the first exercise risk score<$\lambda\cdot$TH, the exercise prompt information is that exercise is recommended.

When TH>the first exercise risk score>$\lambda\cdot$TH, the exercise prompt information is an exercise precaution or an optimized exercise scheme.

When the first exercise risk score>TH, the exercise prompt information is that exercise is not recommended, where TH is determined based on basic information of the user and a blood glucose feature that reflects a blood glucose status of the user within a period of time.

In other words, the exercise risk may be quantified based on the physical condition of the user, so that the electronic device can more accurately evaluate the exercise risk of the user, and obtain an appropriate exercise recommendation between different exercise risks, to help the user exercise more scientifically.

With reference to the third aspect, in an implementation, after obtaining the second exercise reference data of the user during the exercise, the method further includes: determining a second exercise risk score based on the second exercise reference data, where the second risk score indicates an exercise risk during the exercise.

Similarly, during the exercise of the user, the exercise risk during the exercise may be quantified based on the physical condition of the user, to learn of an exercise status of the user in real time.

With reference to the third aspect, in an implementation, the second exercise risk score is obtained through calculation according to the following formula:

$$G=\gamma(t) \sum c_i z_i.$$

Herein, $z_i$ represents a level of an $i^{th}$ piece of exercise reference data in the second exercise reference data, $c_i$ represents a weight of the $i^{th}$ piece of exercise reference data, and $\gamma(t)$ represents a time attenuation factor, where the time attenuation factor is a function that monotonically increases with time t, a larger deviation of a data value of the $i^{th}$ piece of exercise reference data from a normal range indicates a higher level of the $i^{th}$ piece of exercise reference data, a higher second exercise risk score indicates a higher exercise risk, and a lower second exercise risk score indicates a lower exercise risk.

During the exercise of the user, the exercise risk score is calculated based on the time attenuation factor. The time attenuation factor may be used to simulate a delayed hyperglycemia/hypoglycemia risk that gradually increases with time, so that the exercise risk score during the exercise of the user can more accurately and truly reflect the exercise risk of the user.

With reference to the third aspect, in an implementation, after outputting the exercise alarm, the method further includes: outputting the exercise alarm again after fourth duration starting when the user ends the exercise.

In this way, after the exercise ends, the user can be reminded to supplement food, take medicine, and the like in time, to avoid a hyperglycemia phenomenon caused by the exercise and prevent the user from having a delayed hypoglycemia symptom.

With reference to the third aspect, in an implementation, after the user ends the exercise, the method further includes: obtaining exercise feedback information of the user; and outputting the exercise evaluation report of the exercise of the user based on the third exercise reference data specifically includes: outputting the exercise evaluation report of the exercise based on the exercise feedback information and the third exercise reference data, where the exercise evaluation report may further include a recommendation for next exercise that is determined based on the exercise feedback information.

The exercise status of the user is evaluated based on feedback data of the user, so that content in the exercise evaluation report output after the exercise ends can be more comprehensive and richer.

With reference to the third aspect, in an implementation, outputting the exercise evaluation report of the exercise of the user based on the third exercise reference data specifically includes: performing weighted summation on levels of all data in the third exercise reference data to calculate an exercise impact score, where a larger deviation of a data value of data in the third exercise reference data from a normal range indicates a higher level of the data, a higher exercise impact score indicates greater impact of the exercise on the user, and a lower exercise impact score indicates smaller impact of the exercise on the user.

In other words, when the impact of the exercise on the user is evaluated, a plurality of pieces of data may be comprehensively considered for calculation, to evaluate the impact of the exercise on the user as comprehensively and accurately as possible.

According to a fourth aspect, an embodiment of this application further provides a blood glucose management method. The method is applied to an electronic device, and the method includes: first identifying a first exogenous event by using an event identification model, or predicting the first exogenous event based on a work and rest rule of a user, where the event identification model is obtained through training based on a historical exogenous event and a historical blood glucose value of the user, and the first exogenous event may include one or more of the following: a diet event, a medication event, and an exercise event; obtaining blood glucose values of the user within fifth duration before a current time point; and finally predicting blood glucose values within sixth duration after the current time point based on the first exogenous event and the blood glucose values within the fifth duration.

According to the method provided in this embodiment of this application, a future blood glucose value of the user can be predicted based on a blood glucose value of the user and an exogenous event, and external factors that can affect a blood glucose concentration of the user can be considered as much as possible, to improve blood glucose prediction precision and highlight an association between the exogenous event and blood glucose of the user. In this way, the user can better plan a healthy exogenous event to effectively avoid harm caused by hyperglycemia or hypoglycemia. In addition, the user does not need to manually enter the exogenous event, can identify, by using the event identification model, the exogenous event that has occurred on the user, and can also predict an exogenous event that is to occur based on the work and rest rule of the user. This facilitates an operation of the user, and avoids troubles of searching, and an incorrect or missing input of the user, so that a blood glucose prediction technology is more intelligent and comprehensive.

With reference to the fourth aspect, in an implementation, predicting the blood glucose values within the sixth duration after the current time point based on the first exogenous event and the blood glucose values within the fifth duration specifically includes: predicting the blood glucose values within the sixth duration after the current time point by using a blood glucose prediction model and based on the first exogenous event and the blood glucose values within the fifth duration, where an input of the blood glucose prediction model is the exogenous event and the historical blood glucose value, an output is the future blood glucose value, the blood glucose prediction model is obtained through training based on known blood glucose values within a historical first time period, a known exogenous event within the first time period, and known blood glucose values within a historical second time period, and the first time period is a time period adjacent to and before the second time period.

In other words, the future blood glucose value of the user may be predicted by using the blood glucose prediction model, and an association between blood glucose before the exogenous event and blood glucose after the exogenous event and impact of the exogenous event on the blood glucose are fully used, to quickly and accurately predict the future blood glucose.

With reference to the fourth aspect, in an implementation, the event identification model includes an exercise identification model, a medication identification model, and a diet identification model. The exercise identification model is used to identify exercise start time, an exercise volume, and an exercise type of the exercise event, the medication identification model is used to identify medication start time, a medication volume, and a medicine type of the medication event, and the diet identification model is used to identify meal start time, a meal volume, and a diet type of the diet event. If the first exogenous event includes a first exercise event obtained through identification by using the exercise identification model, when the blood glucose prediction model is used to predict a blood glucose value, the input of the blood glucose prediction model includes exercise start time, an exercise volume, and an exercise type of the first exercise event. If the first exogenous event includes a first medication event obtained through identification by using the medication identification model, when the blood glucose prediction model is used to predict a blood glucose value, the input of the blood glucose prediction model includes medication start time, a medication volume, and a medicine type of the first medication event. If the first exogenous event includes a first diet event obtained through identification by using the diet identification model, when the blood glucose prediction model is used to predict a blood glucose value, the input of the blood glucose prediction model includes meal start time, a meal volume, and a diet type of the first diet event.

With reference to the fourth aspect, in an implementation, the method further includes: The electronic device may display a fourth blood glucose curve, where the fourth blood glucose curve may indicate a blood glucose change of the user within a period of time before the current time point, and a blood glucose change obtained through prediction within a period of time after the current time point based on the first exogenous event and the blood glucose values within the fifth duration.

In other words, the electronic device may display known blood glucose and predicted blood glucose of the user through an interaction interface, so that the user learns of a past and future blood glucose change trend, manages and controls a blood glucose change of the user, and avoids in time harm caused by hyperglycemia or hypoglycemia.

With reference to the fourth aspect, in an implementation, the method further includes: The electronic device may further mark the first exogenous event on the fourth blood glucose curve.

In other words, in addition to displaying the known blood glucose and the predicted blood glucose of the user, the electronic device may further mark the exogenous event of the user, and associate the blood glucose of the user with the exogenous event, so that the user controls, in time starting from the exogenous event, a blood glucose change of the user. This helps the user perform the exogenous event in a more scientific and healthier manner and improve a self-management capability of the user on blood glucose.

With reference to the fourth aspect, in an implementation, the method further includes: adjusting the first exogenous event based on a user operation; repredicting blood glucose values within the sixth duration based on an adjusted first exogenous event and the blood glucose values within the fifth duration; and displaying a fifth blood glucose curve, where the fifth blood glucose curve indicates the blood glucose change of the user within the period of time before the current time point, and a blood glucose change obtained through reprediction within the period of time after the current time point.

In other words, the user may manually adjust the exogenous event. The electronic device may synchronously display a blood glucose curve obtained through prediction after the exogenous event is adjusted, to enhance participation of the user, and may further dynamically display an association between the exogenous event and blood glucose. When the user needs to plan a healthy exogenous event, the exogenous event may be adjusted on a premise that the future blood glucose fluctuates within a normal range, and the exogenous event obtained through adjustment is used as the healthy exogenous event planned by the user, to help the user more easily manage and control the blood glucose change of the user.

With reference to the fourth aspect, in an implementation, before identifying the first exogenous event by using the event identification model, or predicting the first exogenous event based on the work and rest rule of the user, the method further includes: displaying a sixth blood glucose curve, where the sixth blood glucose curve indicates the blood glucose change of the user within the period of time before the current time point, and a blood glucose change obtained through prediction within the period of time after the current time point based on the blood glucose values within the fifth duration.

In this way, both the blood glucose curve obtained through prediction without considering the exogenous event and the blood glucose curve obtained through prediction when the exogenous event is considered may be displayed, to highlight a difference between blood glucose predictions obtained by considering the exogenous event and without considering the exogenous event in this method.

With reference to the fourth aspect, in an implementation, before identifying the first exogenous event by using the event identification model, the method further includes: determining that a blood glucose change rate of the user within seventh duration before the current time point is greater than a fifth threshold.

In this way, the exogenous event that has occurred on the user may be obtained only when it is determined that blood glucose data of the user meets the preset condition, and the future blood glucose may be predicted by using the exogenous event and the blood glucose data.

According to a fifth aspect, an embodiment of this application provides an electronic device, including a memory, one or more processors, and one or more programs. When the one or more processors execute the one or more programs, the electronic device is enabled to implement the method described in any one of the first aspect or the implementations of the first aspect, any one of the second aspect or the implementations of the second aspect, any one of the third aspect or the implementations of the third aspect, or any one of the fourth aspect or the implementations of the fourth aspect.

According to a sixth aspect, an embodiment of this application provides a computerreadable storage medium, including instructions. When the instructions are run on an electronic device, the electronic device is enabled to perform the method described in any one of the first aspect or the implementations of the first aspect, any one of the second aspect or the implementations of the second aspect, any one of the third aspect or the implementations of the third aspect, or any one of the fourth aspect or the implementations of the fourth aspect.

According to a seventh aspect, an embodiment of this application provides a computer program product. When the computer program product runs on a computer, the computer is enabled to perform the method described in any one of the first aspect or the implementations of the first aspect, any one of the second aspect or the implementations of the second aspect, any one of the third aspect or the implementations of the third aspect, or any one of the fourth aspect or the implementations of the fourth aspect.

**BRIEF DESCRIPTION OF DRAWINGS**

FIG. 1 is a diagram of a communication system 1000 according to an embodiment of this application;

FIG. 2 is a schematic flowchart of a blood glucose management method according to an embodiment of this application;

FIG. 3 is an example diagram of a blood glucose curve of a user within 24 hours according to an embodiment of this application;

FIG. 4 is an example diagram of blood glucose curves formed by connecting key blood glucose values in a diet event of a user within one month according to an embodiment of this application;

FIG. 5 is an example diagram of a blood glucose curve of a user within 24 hours according to an embodiment of this application;

FIG. 6 is a schematic flowchart of another blood glucose management method according to an embodiment of this application;

FIG. 7 is a schematic flowchart of a method for identifying that a user starts a meal according to an embodiment of this application;

FIG. 8(a) to FIG. 8(d) are a diagram of a process in which two blood glucose curves are normalized into a standard blood glucose curve according to an embodiment of this application;

FIG. 9 is a schematic flowchart of evaluating an exercise risk of a user before exercise according to an embodiment of this application;

FIG. 10 is a schematic flowchart of evaluating an exercise risk of a user during exercise according to an embodiment of this application;

FIG. 11 is a schematic flowchart of evaluating an exercise risk of a user after exercise according to an embodiment of this application;

FIG. 12 is a schematic flowchart of another blood glucose management method according to an embodiment of this application;

FIG. 13 is a diagram of blood glucose curves in a case in which an exogenous event is an event that has occurred currently according to an embodiment of this application;

FIG. 14 is a diagram of blood glucose curves in a case in which an exogenous event is an event that has not occurred currently according to an embodiment of this application;

FIG. 15 is a diagram of a hardware structure of an electronic device 100 according to an embodiment of this application;

FIG. 16 is a block diagram of a software structure of an electronic device 100 according to an embodiment of this application;

FIG. 17 is a diagram of a hardware structure of an electronic device 200 according to an embodiment of this application; and

FIG. 18 is a diagram of a hardware structure of an electronic device 300 according to an embodiment of this application.

**DESCRIPTION OF EMBODIMENTS**

The technical solutions in embodiments of this application are clearly and completely described in the following with reference to the accompanying drawings. In the descriptions of embodiments of this application, unless otherwise stated, "/" represents "or". For example, A/B may represent A or B. In this specification, "and/or" merely describes an association relationship between associated objects and represents that three relationships may exist. For example, A and/or B may represent the following three cases: Only A exists, both A and B exist, and only B exists. In addition, in the descriptions of embodiments of this application, "a plurality of" means two or more than two.

Terms "first" and "second" mentioned below are merely intended for description, and shall not be understood as an indication or implication of relative importance or implicit indication of a quantity of indicated technical features. Therefore, a feature limited by "first" or "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form that can be accepted by the user. The user interface is source code written in a specific computer language like Java or an extensible markup language (extensible markup language, XML). Interface source code is parsed and rendered on an electronic device, and is finally presented as content that can be identified by the user. The user interface is usually represented in a form of a graphical user interface (graphical user interface, GUI), and is a user interface that is displayed in a graphical manner and that is related to a computer operation. The user interface may be a visual interface element like a text, an icon, a button, a menu, a tab, a text box, a dialog box, a status bar, a navigation bar, or a widget that is displayed on a display of the electronic device.

Diabetes is a metabolic disease characterized by hyperglycemia. Long-term hyperglycemia may lead to chronic damage and dysfunction of various tissues, particularly eyes, kidneys, heart, blood vessels, and nerves. It can be learned that effective diabetes management requires real-time monitoring of blood glucose concentration of patients. Exogenous events such as an exercise event, a diet event, and a medication event affect blood glucose changes of the patients. Therefore, monitoring of the exogenous events is vital for controlling the blood glucose concentrations of the patients.

Therefore, how to use a blood glucose value of a user to help the user better manage and control impact of an exogenous event on blood glucose is an urgent problem to be resolved currently.

According to a first aspect, an embodiment of this application provides a blood glucose management method. In the method, meal time of a diet event of a user can be determined based on a blood glucose value of the user, and when a blood glucose curve drawn based on blood glucose values of the user is displayed, the meal time and/or a blood glucose value related to the meal time are/is displayed on the blood glucose curve.

In this way, the user may learn of a change trend of a blood glucose concentration of the user within a period of time by using the blood glucose curve. In addition, the diet event of the user is associated with blood glucose of the user, and a blood glucose change of the user before and after a meal is highlighted. This helps the user more intuitively learn of impact of the diet event on the blood glucose, and helps the user better manage and control the blood glucose concentration. In addition, for a diabetic patient, a self-management capability of the user for diabetes can be further enhanced.

According to a second aspect, an embodiment of this application further provides a blood glucose management method. In the method, an actual blood glucose curve drawn based on blood glucose values of a user during a meal can be obtained, a meal volume and a meal speed of the user during the meal can be calculated, a standard blood glucose curve of healthy people who have the same meal volume and meal speed is obtained, and diet health of the user is evaluated by comparing the actual blood glucose curve with the standard blood glucose curve.

In this way, a difference between blood glucose of the user and normal blood glucose of the healthy people can be quantified based on a meal status of the user, to help the user evaluate, based on the blood glucose value during the meal, whether a blood glucose response of the user to a diet is healthy, and better learn of a physical condition of the user.

According to a third aspect, an embodiment of this application further provides a blood glucose management method. In the method, before a user exercises, an exercise risk score of the user in the exercise can be calculated based on exercise reference data of the user before the exercise, and corresponding exercise prompt information can be output based on a value of the exercise risk score. In addition, in the method, when the user exercises, an exercise risk score of the user during the exercise can be further calculated based on exercise reference data of the user during the exercise, and it is determined, based on the exercise risk score, whether to output an exercise alarm and interrupt the exercise of the user. Finally, in the method, after the exercise of the user ends, an exercise evaluation report of the exercise can be further output based on exercise reference data of the exercise of the user. The exercise reference data includes blood glucose of the user, a parameter reflecting a vital sign of the user, and the like.

It can be learned that, in the method, an exercise risk of the user can be evaluated based on data related to the exercise of the user in an entire process from a moment before the user exercises to a moment after the user exercises, to avoid as much as possible that excessively high or low blood glucose harms health of the user, and help the user exercise in a healthier and safer way.

According to a fourth aspect, an embodiment of this application further provides a blood glucose management method. In the method, an exogenous event can be identified by using an event identification model, or the exogenous event can be predicted based on a work and rest rule of a user. The event identification model is obtained through training based on a historical exogenous event and a historical blood glucose value of the user, and the exogenous event may include one or more of the following: a diet event, a medication event, and an exercise event. Then, blood glucose data within a future period of time is predicted based on the exogenous event and blood glucose data within a period of time before a current time point.

It can be learned that, the exogenous event that can affect a blood glucose change of the user is used as a factor for predicting blood glucose, so that accuracy of predicting future blood glucose can be improved, and an association between blood glucose of the user and the exogenous event can be highlighted, so that the user can better manage and control the blood glucose change of the user starting from the exogenous event and by adjusting the exogenous event, to avoid harm caused by excessively high or low blood glucose. In addition, the exogenous event does not need to be manually entered by the user. This facilitates an operation of the user and improves user experience.

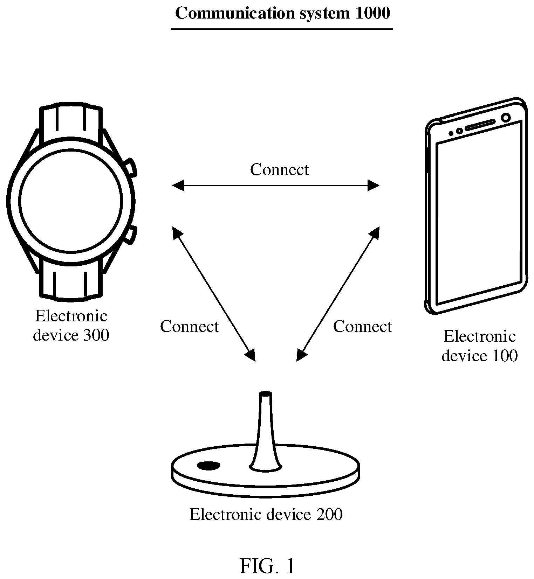

**FIG. 1 is a diagram of a communication system 1000 according to an embodiment of this application.**

In this embodiment of this application, the communication system 1000 may include a display device and a data collection device.

The display device may display text and/or image information used to reflect a blood glucose status of a user, for example, an exercise risk score of the user, a diet, exercise, and a medication recommendation, and a blood glucose graph of the user, where the blood glucose graph may include a blood glucose curve formed by connecting blood glucose values of the user within a period of time. The data collection device may be configured to collect data such as a blood glucose value, a heart rate, a breath, a body exercise frequency, a body exercise direction, a body exercise range, and a quantity of exercise times of the user.

The display device may include one or more devices. The display device may be a device including a display, like a mobile phone, a computer, a tablet, or a wearable device. The data collection device may include one or more devices. The data collection device may collect data by using a sensor included in the data collection device, or may obtain data entered by the user or generate data by using the data collection device. When the data collection device includes a plurality of devices, different data collection devices may be configured to collect different data of the user. For example, a device for collecting a blood glucose value of the user may be a continuous glucose monitoring (continuous glucose monitoring, CGM) device. The CGM device may implant a biosensor (a microneedle sensor) subcutaneously to be in contact with a tissue fluid, to determine a tissue fluid glucose concentration, and then obtain the blood glucose value by compensating for a delay between tissue fluid glucose and blood glucose.

It may be understood that the communication system 1000 may further include another device, for example, a storage device. The storage device may be configured to store the data collected by the data collection device, the text and/or image information displayed by the display device, and the like. For example, the storage device may be a server.

For example, the communication system 1000 shown in FIG. 1 includes an electronic device 100, an electronic device 200, and an electronic device 300. As shown in FIG. 1, the electronic device 100 may be a mobile phone, the electronic device 200 may be a CGM device, and the electronic device 300 may be a smartwatch. The electronic device 100 may be the display device mentioned above, and the electronic device 200 and the electronic device 300 may be data collection devices mentioned above.

A communication connection may be established between any two of the electronic device 100, the electronic device 200, and the electronic device 300. Specifically, the communication connection may be a wired connection or a wireless connection. The wireless connection may be a short-distance connection like a high-fidelity wireless communication (wireless fidelity, Wi-Fi) connection, a Bluetooth connection, an infrared connection, an NFC connection, or a ZigBee connection, or may be a long-distance connection. The long-distance connection includes but is not limited to a long-distance connection based on 2G, 3G, 4G, 5G, and mobile networks of subsequent standard protocols. For example, the electronic device 100 may establish a communication connection to the electronic device 300 through Bluetooth.

In the communication system 1000 shown in FIG. 1, the electronic device 200 may collect the blood glucose value of the user, and send the blood glucose value to the electronic device 100. The electronic device 300 may be configured to: collect the data such as the heart rate, the breath, the body exercise frequency, the body exercise direction, the body exercise range, and the quantity of exercise times of the user, and send the data to the electronic device 100. The electronic device 100 processes the obtained data, and displays the text and/or image information used to reflect the blood glucose status of the user.

It may be understood that the communication system 1000 shown in FIG. 1 may include more or fewer devices. For example, the communication system 1000 may include only the electronic device 200 and the electronic device 300. The electronic device 200 may send the collected blood glucose value of the user to the electronic device 300, and the electronic device 300 may display, based on the data collected by the electronic device 300 and the received blood glucose value, the text and/or image information used to reflect the blood glucose status of the user. In this case, the electronic device 200 may be the display device and the data collection device that are mentioned above, and the electronic device 200 may be the data collection device mentioned above.

It should be noted that, in the communication system 1000, one device may be a display device or a data collection device, or one device may be both a display device and a data collection device. This is not limited in embodiments of this application.

To help a user better manage and control impact of an exogenous event on blood glucose, this application provides a plurality of blood glucose management methods based on a blood glucose value of the user, to help the user effectively control a blood glucose change as much as possible in daily life, and ensure a healthy life of the user.

The following separately describes the plurality of blood glucose management methods by using Embodiment 1 to Embodiment 4.

**Embodiment (1)**

To monitor a blood glucose change of a user in real time, and help the user adjust blood glucose in time and control a hazard of diabetes, a blood glucose curve of the user within a period of time may be presented to the user, to reflect a blood glucose status of the user within the period of time.

However, a diet status of the user is not marked on the blood glucose curve, or only a diet event of the user is roughly marked on the blood glucose curve. However, food intake directly affects a blood glucose concentration change of the user. Displaying only the blood glucose curve or displaying only fuzzy information of the diet event on the blood glucose curve cannot well help the user intervene or manage and control the blood glucose change and effectively control impact of diabetes on user health.

This embodiment of this application provides a blood glucose management method. In the method, meal time of the diet event of the user can be calculated based on a blood glucose value of the user, and the meal time and/or a blood glucose value related to the meal time are/is displayed on the blood glucose curve. In this way, the user can associate blood glucose with the diet event, to better learn of a blood glucose change before and after a meal, so that the user can intervene and control the blood glucose concentration change starting from a diet.

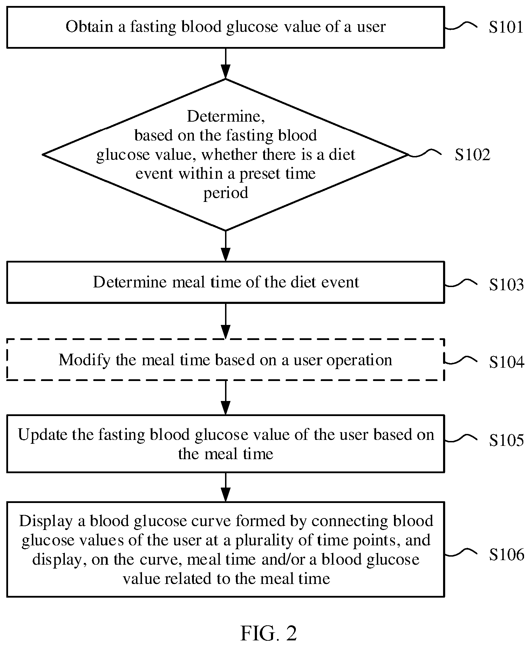

**FIG. 2 is a schematic flowchart of a blood glucose management method according to this embodiment of this application.**

As shown in FIG. 2, the method includes the following steps.

**S101:** Obtain a fasting blood glucose value of the user.

The fasting blood glucose value is a blood glucose value of the user on an empty stomach.

The fasting blood glucose value of the user may be obtained in any one of the following manners:

(1) Determine the fasting blood glucose value based on blood glucose values of the user within a period of time.

For example, the blood glucose values of the user within the period of time may be collected by using a CGM device, for example, the electronic device 200. The blood glucose values within the period of time include blood glucose values at a plurality of time points within the period of time.

A specific example determining method for determining the fasting blood glucose value based on the blood glucose values may include but is not limited to the following three methods:

(a) Determine a lower quartile of the blood glucose values collected within the period of time as the fasting blood glucose value of the user.

For example, the period of time may be 24 hours. In other words, the blood glucose values of the user collected within the 24 hours are sorted in ascending order, and a blood glucose value ranked in a quarter is the fasting blood glucose value of the user.

(b) Determine a lower quartile of blood glucose values between two meals of the user within the period of time as the fasting blood glucose value of the user.

For example, the period of time may be 24 hours, and the two meals may be the last meal on the previous day and the first meal on the next day within the 24 hours. In other words, the blood glucose values of the user collected within a time period from the last meal on the previous day to the first meal on the next day are sorted in ascending order, and a blood glucose value ranked in a quarter is the fasting blood glucose value of the user.

(c) Determine a smaller value in the values obtained in (a) and (b) as the fasting blood glucose value of the user.

It can be learned that, because the fasting blood glucose value is the blood glucose value of the user on an empty stomach, the lower quartile of the blood glucose values collected within the period of time is determined as the fasting blood glucose value of the user, so that impact of excessively high or low blood glucose on the fasting blood glucose value can be eliminated, and the fasting blood glucose value of the user is directly and accurately obtained based on the blood glucose values of the user within the period of time.

In addition, it may be understood that the period of time may be a latest period of time that currently satisfies a requirement. For example, 24 hours in the method a are 24 hours backtracked from a current time point. In this embodiment of this application, the period of time may alternatively be referred to as a second time period.

(2) Obtain a fasting blood glucose value determined when meal time is determined last time.

Because the blood glucose management method provided in this embodiment of this application may be repeatedly performed, the user may determine meal time within different time periods by using the method a plurality of times. Therefore, when meal time (for example, first meal time) within a period of time is determined this time, a fasting blood glucose value determined when meal time (for example, second meal time) within another period of time is determined last time may be used.

Specifically, the fasting blood glucose value determined based on the meal time when the meal time is determined last time may be directly determined as a fasting blood glucose value that needs to be obtained before the meal time is determined this time. In other words, when the meal time is determined by using the blood glucose management method this time, the fasting blood glucose value that needs to be obtained in step S101 is a fasting blood glucose value determined by using step S105 when the meal time is determined by using the blood glucose management method last time.

For details about the fasting blood glucose value determined based on the meal time, refer to subsequent step S105. Details are not described herein.

In some implementations, the manner 1 of obtaining the fasting blood glucose value may be applied to a process of determining the fasting blood glucose value for the first time, and the manner 2 of obtaining the fasting blood glucose value is applied to a process of determining the fasting blood glucose value not for the first time. In other words, in a process in which the user continuously determines meal time a plurality of times, when the user determines the meal time by using the blood glucose management method for the first time, blood glucose values of the user within a period of time may be collected, and the fasting blood glucose value of the user is determined by using these blood glucose values. Therefore, meal time of a diet event of the user is determined by using the fasting blood glucose value, and the fasting blood glucose value of the user is updated based on the meal time. Then, when the user redetermines meal time of the user, an updated fasting blood glucose value may be used to determine meal time of a diet event of the user within a next period of time, and the fasting blood glucose value is updated based on the meal time. By analogy, an updated fasting blood glucose value is used to determine meal time.

It may be understood that the obtained fasting blood glucose value may alternatively be a smaller value in the fasting blood glucose values obtained in the foregoing manner 1 and manner 2. A manner of obtaining the fasting blood glucose value is not limited in embodiments of this application.

**S102:** Determine, based on the fasting blood glucose value, whether there is a diet event within a preset time period.

Whether blood glucose values within the preset time period meet a preset condition may be determined based on the fasting blood glucose value. If the blood glucose values meet the preset condition, it is determined that there is a diet event within the preset time period; otherwise, there is no diet event within the preset time period.

For example, the preset condition may be that the blood glucose values within the preset time period (for example, a first time period) exceed the fasting blood glucose value by a threshold (for example, a first threshold). For example, the preset time period may be a time period whose time length is one hour. Further, the preset time period may be a time period in which one hour is backtracked from the current time point. The threshold may be 2 mmol/L.

It may be understood that the time length of the preset time period may alternatively be another value, for example, two hours. This is not limited in embodiments of this application. Alternatively, the threshold may be another value. For example, the threshold may be adaptively modified based on different users. This is because different users have different physical conditions, and consequently, relative fasting blood glucose values of the different users are different. The threshold is not limited in embodiments of this application.

When it is determined, based on the fasting blood glucose value, that there is a diet event within the preset time period, step S103 is performed.

**S103:** Determine meal time of the diet event.

The meal time may be a time period, or may be a time point. This is not limited in embodiments of this application.

This embodiment of this application provides two manners of determining the meal time.

(1) The meal time of the diet event may be determined based on the blood glucose values within the preset time period.

The meal time may be backtracked from a blood glucose peak within the preset time period.

Specifically, determining the meal time may include: determining a time point (referred to as a first time point below) of the blood glucose peak within the preset time period, and backtracking preset duration (for example, second duration) from the time point to find another time point (referred to as a second time point below). The meal time is between the first time point and the second time point. Blood glucose values between the first time point and the second time point are obtained. A blood glucose value $G_t$ at meal time t is a blood glucose value at an earliest time point in blood glucose values that are between the two time points and that satisfy the following Formula 1:

$$G_t > G_0 \text{ and } \frac{G_{t+\Delta t} - G_t}{\Delta t} > 0.06 \qquad \text{Formula 1}$$

$G_0$ represents the fasting blood glucose value, $\Delta t$ represents an interval for collecting a blood glucose value, t represents the meal time, the blood glucose value $G_t$ represents a blood glucose value of the user collected at the meal time, and $G_{t+\Delta t}$ represents a blood glucose value of the user collected last time after the meal time.

It can be learned from Formula 1 that, an interval between the meal time and the time point of the blood glucose peak is less than preset duration (for example, three hours), the blood glucose value at the meal time is greater than the fasting blood glucose value, and a blood glucose rising rate at the meal time is greater than 0.06.

It may be understood that the blood glucose rising rate at the meal time may alternatively be greater than another threshold. The threshold is not limited in embodiments of this application. The threshold may alternatively be referred to as a second threshold in this embodiment of this application.

(2) The meal time of the diet event may be determined based on image shooting time at which the user shoots food.

In other words, an image shooting time point at which the user shoots food is the meal time of the diet event.

During specific implementation, in a process in which the user takes a photo, if it is identified that a shot object in a shot image is food, an image shooting time point at which a photo is taken is recorded. Further, when it is determined, based on the fasting blood glucose value, that there is a diet event within preset time, the image shooting time point is determined as the meal time of the diet event.

It may be understood that a manner of determining the meal time is not limited in embodiments of this application. For example, the meal time may alternatively be entered by the user. This is not limited in embodiments of this application.

S104: Modify the meal time based on a user operation.

In other words, after the meal time is obtained through calculation based on the known blood glucose values of the user, the user may further manually modify the meal time, and correct the meal time, to ensure accuracy of the meal time of the user.

It may be understood that step S104 is an optional step.

S105: Update the fasting blood glucose value of the user based on the meal time.

For example, an updated fasting blood glucose value of the user may be an average value of blood glucose values collected within preset duration (for example, 30 minutes) before the meal time.

In this embodiment of this application, the preset duration may alternatively be referred to as third duration.

S106: Display a blood glucose curve formed by connecting blood glucose values of the user at a plurality of time points, and display, on the blood glucose curve, meal time and/or a blood glucose value related to the meal time.

The blood glucose curve may include a blood glucose curve (for example, a first blood glucose curve) that includes the preset time period and that is formed by connecting blood glucose values collected at a plurality of consecutive time points. The blood glucose curve may indicate a blood glucose change of the user within the preset time period.

For example, the blood glucose curve that includes the preset time period may be a blood glucose curve formed by connecting blood glucose values with a time span of 24 hours. The blood glucose value related to the meal time may include but is not limited to a blood glucose value at the meal time, and a blood glucose value at a time point preset duration (for example, first duration) before or after the meal time, for example, a blood glucose value one hour after the meal time, a blood glucose value two hours after the meal time, or a blood glucose value three hours after the meal time.

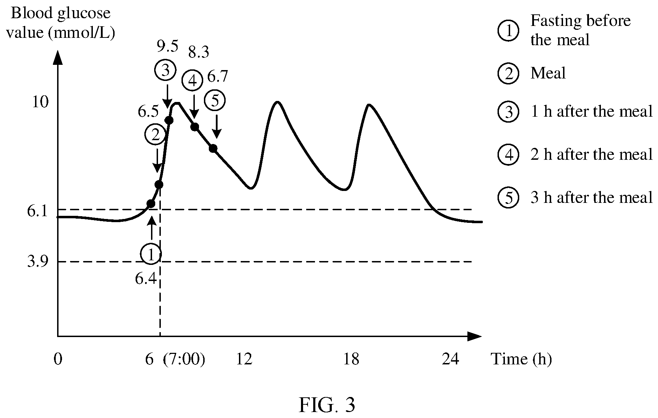

FIG. 3 is an example diagram of a blood glucose curve of the user within 24 hours according to this embodiment of this application.

As shown in FIG. 3, a normal fasting blood glucose value is 3.9 mmol/L to 6.1 mmol/L. The blood glucose curve further shows that a fasting blood glucose value of the user before breakfast is 6.4 mmol/L, meal time of the user during breakfast is 7:00, a blood glucose value of the user during breakfast is 6.5 mmol/L, a blood glucose value obtained one hour after breakfast is 9.5 mmol/L, a blood glucose value obtained two hours after breakfast is 8.3 mmol/L, and a blood glucose value obtained three hours after breakfast is 6.7 mmol/L.

It can be learned from FIG. 3 that, the user can learn of a blood glucose concentration change of the user within 24 hours by using the blood glucose curve, and a blood glucose value at a key time point of a meal event is marked, so that the user can easily identify blood glucose values before and after a meal. In this way, the user can promptly provide a coping strategy for an abnormal blood glucose change during a diet of the user.

In addition, the blood glucose curve may further include the following: in a diet event (for example, a breakfast event) on each day in a preset quantity of days (for example, N days, where N≥1, and N is a positive integer), a blood glucose curve (for example, a second blood glucose curve) formed by connecting fasting blood glucose values, a blood glucose curve formed by connecting blood glucose values at meal time points, and a blood glucose curve (for example, a third blood glucose curve) formed by connecting blood glucose values at time points preset duration before or after the meal time point, that is, a blood glucose curve formed by connecting blood glucose values at preset time points before or after a meal, for example, a blood glucose curve formed by connecting blood glucose values one hour after the meal, a blood glucose curve formed by connecting blood glucose values two hours after the meal, or a blood glucose curve formed by connecting blood glucose values three hours after the meal.

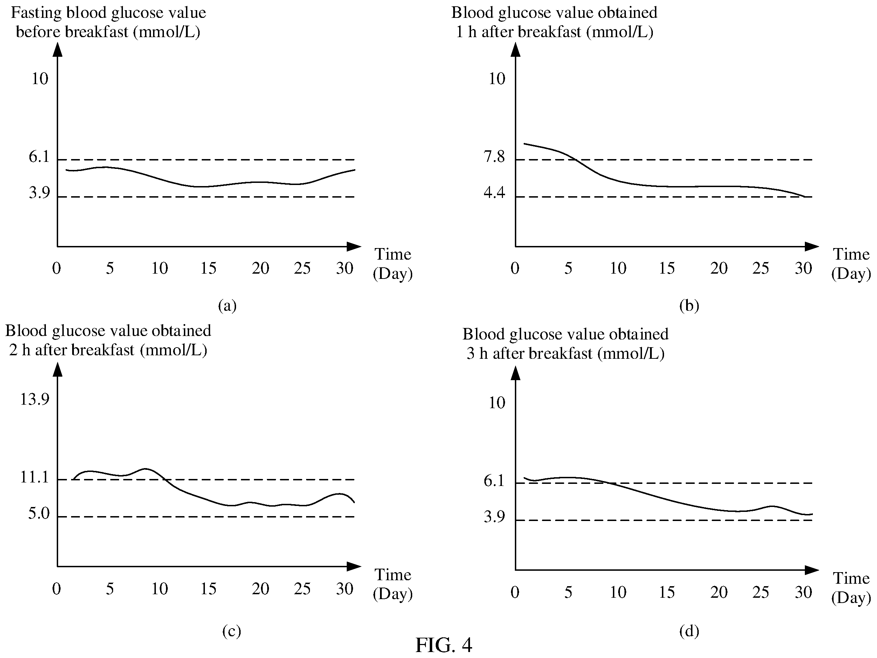

For example, the preset quantity of days is one month. FIG. 4 is an example diagram of blood glucose curves formed by connecting key blood glucose values in a diet event of the user within one month according to this embodiment of this application.

(a) in FIG. 4 shows an example of a blood glucose curve formed by connecting fasting blood glucose values in a same diet event of the user within one month, (b) in FIG. 4 shows an example of a blood glucose curve formed by connecting blood glucose values one hour after a meal in a same diet event of the user within one month, (c) in FIG. 4 shows an example of a blood glucose curve formed by connecting blood glucose values two hours after the meal in a same diet event of the user within one month, and (d) in FIG. 4 shows an example of a blood glucose curve formed by connecting blood glucose values three hours after the meal in a same diet event of the user within one month.

A change of fasting blood glucose of the user before the meal for one month can be learned from (a) in FIG. 4, a change of the blood glucose values of the user 1 h after the meal for one month can be learned from (b) in FIG. 4, a change of the blood glucose values of the user 2 h after the meal for one month can be learned from (c) in FIG. 4, and a change of the blood glucose values of the user 3 h after the meal for one month can be learned from (d) in FIG. 4. It can be learned that FIG. 4 can help the user view, from a long-term perspective, a blood glucose change of the user within a period of time and an association between the diet event and a blood glucose change, so that the user can make an overall dietary adjustment to cope with an abnormal blood glucose change.

In some implementations, in addition to the blood glucose curve, diet information of the diet event may be displayed. For example, the diet information may include but is not limited to one or more of the following: a food type, total calories of food, a proportion of each nutrient, and the like. The diet information may be obtained by identifying, by using an artificial intelligence (Artificial Intelligence, AI) algorithm, an image shot by the user for the diet event, or may be obtained through entering by the user. The diet information and a manner of obtaining the diet information are not limited in embodiments of this application.

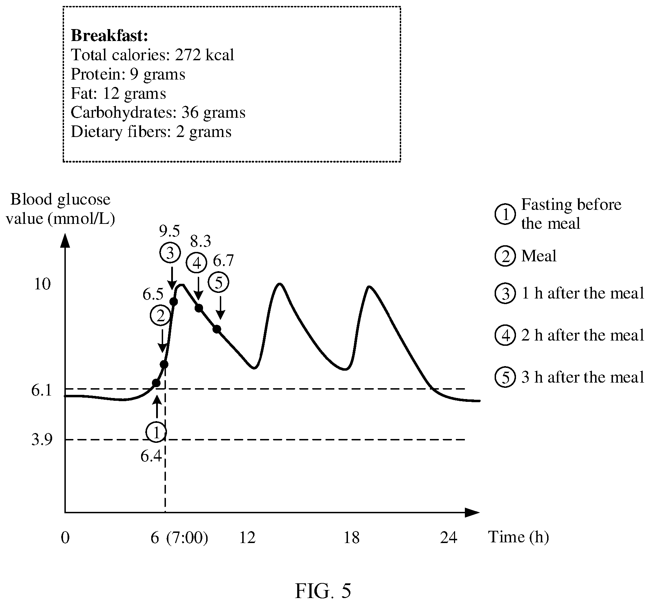

FIG. 5 is an example diagram of a blood glucose curve of the user within 24 hours according to this embodiment of this application.

In comparison with FIG. 3, in FIG. 5, in addition to the blood glucose curve and a key blood glucose value on the blood glucose curve, diet information of a breakfast event of the user is displayed, including but not limited to one or more of the following: calories of food, a proportion of each nutrient, and the like. For example, as shown in FIG. 5, the diet information includes: 272 kilocalories of total calories, 9 grams of protein, 12 grams of fat, 36 grams of carbohydrates, and 2 grams of dietary fibers.

It can be learned that, in addition to a blood glucose concentration change of the user within 24 hours and an association between a meal and blood glucose, the user may learn of detailed information of the meal of the user from FIG. 5.

For example, the diagrams of the blood glucose curves shown in FIG. 3, FIG. 4, and FIG. 5 may be displayed by using the electronic device 100 mentioned above. In this way, the user may learn of a blood glucose status of the user by viewing content displayed on the electronic device 100.

It may be understood that step S106 may be performed after step S103. In other words, after the meal time is obtained through calculation based on the known blood glucose values of the user, the blood glucose curve may be displayed, and the blood glucose value related to the meal time is displayed on the blood glucose curve. Further, the user may manually correct the meal time. In this case, the meal time and the blood glucose value related to the meal time that are displayed on the blood glucose curve may be synchronously updated, and the fasting blood glucose value of the user is updated based on the corrected fasting blood glucose value.

In general, according to the blood glucose management method provided in Embodiment 1, the meal time of the user can be obtained through calculation based on the known blood glucose values of the user, to help the user learn of the blood glucose change of the user before and after the meal, and further help the user find a causal relationship between the meal, insulin, and various other events that affect a blood glucose concentration, so that a positive feedback can be formed between a blood glucose concentration change of the user and diet adjustment, to improve an ability of diabetic patients to manage and control diabetes. For example, when the user learns of, based on the blood glucose curve, that hypoglycemia occurs in two to three hours after the meal of the user, the user may consider whether to alleviate the hypoglycemia symptom by adjusting a diet or taking medicine, consider whether meal time or medication time, and a type and quantity of meal and medication need to be adjusted, or the like.

**Embodiment (2)**

Diabetes is a common disease, but only a small quantity of people have professional knowledge of diabetes. For common people, it is vital to evaluate health based on a blood glucose value. For example, a fasting blood glucose value measured by a user is 7.3 mmol/L. If the user does not know a normal fasting blood glucose range, the user cannot determine whether the fasting blood glucose value is normal only based on the fasting blood glucose value.

Therefore, to help the user have a clearer understanding of health of the user by using a blood glucose value, this embodiment of this application provides a blood glucose management method. In the method, a meal volume and a meal speed of the user during a meal can be determined, an actual blood glucose curve of the user during the meal is obtained, and then a standard blood glucose curve of healthy people who have a same meal volume and meal speed is obtained. Diet health of the user can be evaluated based on the actual blood glucose curve and the standard blood glucose curve, a larger difference between the actual blood glucose curve and the standard blood glucose curve indicates an unhealthier diet of the user, and a smaller difference between the actual blood glucose curve and the standard blood glucose curve indicates a healthier diet of the user.

It can be learned that, in the method, a difference between blood glucose of the user and standard blood glucose of the healthy people during the meal can be quantified, and blood glucose health of the user after the meal can be evaluated. In this way, even if the user has no professional knowledge of diabetes, blood glucose, or the like, the user can still easily learn of impact of the meal on the blood glucose of the user, so that the user can intervene or manage and control a blood glucose change.

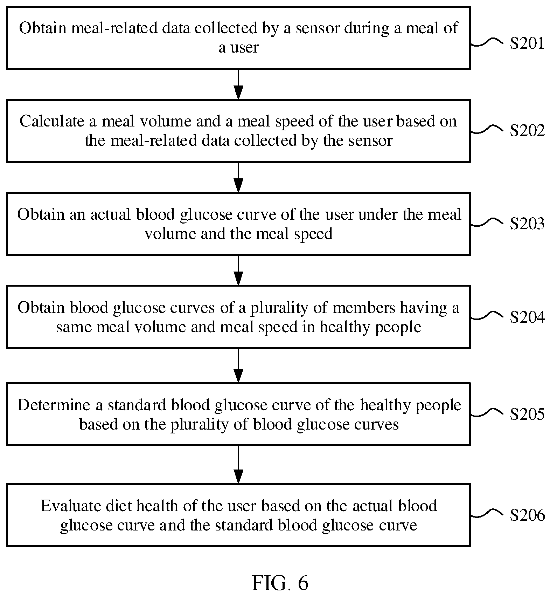

**FIG. 6 is a schematic flowchart of another blood glucose management method according to this embodiment of this application.**

As shown in FIG. 6, the method includes the following steps.

**S201:** Obtain meal-related data collected by a sensor during the meal of the user.

The meal-related data is vital sign data related to the meal of the user. For example, the meal-related data may include but is not limited to heart rate data, exercise data, emotion and stress data, and the like.

For example, the meal-related data of the user may be collected by using a sensor in the electronic device 100 and/or the electronic device 300 mentioned above.

It should be noted that, in a process of collecting the meal-related data by using the sensor, time points at which the meal starts and ends need to be identified and determined, so that the meal-related data during the meal can be obtained.

Identifying that the meal starts may include but is not limited to the following two manners:

(1) Detect that the user confirms an operation of starting the meal.

In this case, the user may actively enter the operation of starting the meal to an electronic device (for example, the electronic device 100), so that the electronic device identifies that the user starts the meal, and collects, by using a data collection device (for example, the electronic device 300), the meal-related data of the user during the meal.

(2) Data collected by the sensor satisfies a preset condition.

In this case, the electronic device may collect all or a part of the meal-related data in real time, and when determining that the all or the part of the data satisfies the preset condition, the electronic device determines that the user starts the meal, and obtains the meal-related data of the user during the meal.

The data collected by the sensor indicates data such as a heart rate, exercise, and emotion and stress of the user. Because the heart rate of the user changes before and after a diet, whether the user starts the meal may be determined based on a difference between the heart rate of the user and a resting heart rate. In addition, because factors such as exercise, and emotion and stress also affect a heart rate change of the user, before it is determined, based on the heart rate of the user, whether the user starts the meal, impact of exercise, and emotion and stress on the heart rate of the user needs to be excluded.

In other words, the preset condition may include that the difference between the heart rate of the user and the resting heart rate is still greater than a threshold (for example, a third threshold) after the impact of the exercise, and the emotion and stress is excluded.

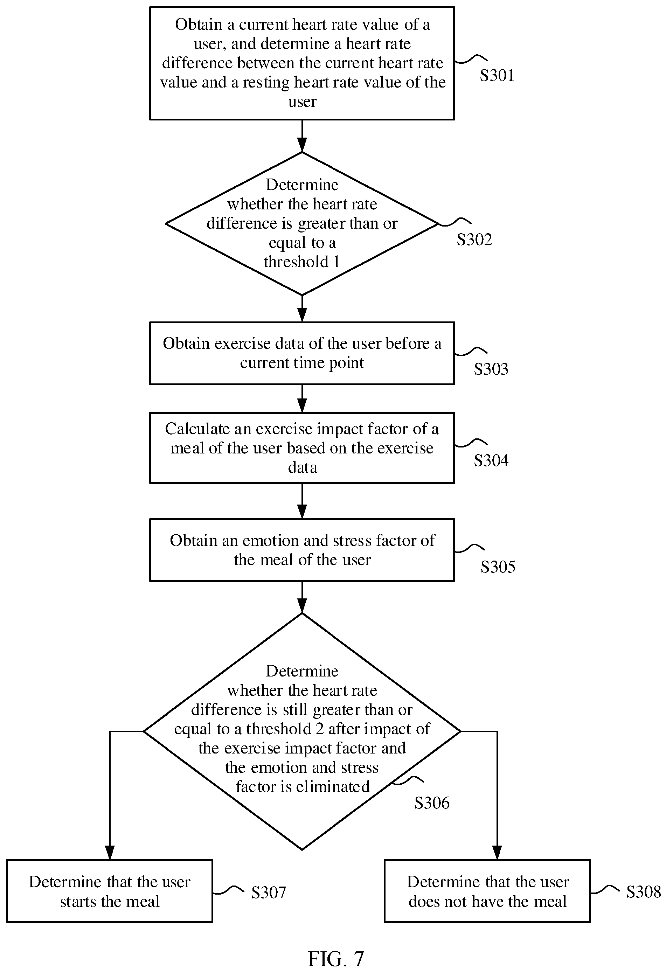

**The following describes, by using FIG. 7, a detailed process of identifying starting a meal based on the data collected by the sensor.**

FIG. 7 is a schematic flowchart of a method for identifying that the user starts the meal according to this embodiment of this application.

As shown in FIG. 7, a detailed process of identifying that the user starts the meal may include the following steps.

**S301:** Obtain a current heart rate value of the user, and determine a heart rate difference between the current heart rate value and the resting heart rate value of the user.

After the user starts the meal, the heart rate value changes. Therefore, whether the user starts the meal may be determined based on the heart rate value of the user.

The heart rate value of the user and the resting heart rate value of the user may be obtained by using a sensor (for example, a bone conduction sensor) in an electronic device (for example, the electronic device 100). The resting heart rate is also referred to as a quiet heart rate, and refers to a heart rate in a quiet, inactive, and awake state.

The heart rate difference between the current heart rate value and the resting heart rate value of the user may be determined by using the following Formula 2:

$$\Delta HR = HR_N - HR_0 \quad \text{Formula 2}$$

Herein, $\Delta HR$ represents the heart rate difference, $HR_N$ represents the current heart rate value of the user, and $HR_0$ represents the resting heart rate value of the user.

**S302:** Determine whether the heart rate difference is greater than or equal to a threshold 1.

When it is determined that the heart rate difference is greater than or equal to the threshold 1 (for example, 10 times or 20 times), step S302 is performed.

**S303:** Obtain exercise data of the user before a current time point.

Exercise data of the user within preset duration before the current time point may be obtained. For example, the preset duration may be 30 minutes, 1 hour, or the like.

In other words, when the difference between the current heart rate value and the resting heart rate value of the user is greater than or equal to the threshold 1, exercise data of the user within a previous period of time is obtained. The exercise data reflects an exercise status of the user, and a heart rate change of the user is affected after the user exercises.

For example, the exercise data of the user may include but is not limited to one or more of the following: an exercise frequency, an exercise speed, an exercise distance, and the like. For example, when exercise performed by the user within the preset duration before the current time point includes walking, the exercise data of the user may include a stride frequency, a stride speed, and a walking distance.

For example, the exercise data of the user may be collected by using a sensor (for example, a gyroscope sensor or an acceleration sensor) in an electronic device (for example, the electronic device 300).

**S304:** Calculate an exercise impact factor of the meal of the user based on the exercise data.

The exercise of the user also leads to the heart rate change of the user. Therefore, to avoid that the exercise of the user affects the heart rate and further affects identification of the meal of the user, before it is determined whether the user starts the meal, impact of the exercise of the user on identification of the meal is first eliminated.

The exercise impact factor may be obtained through calculation by using the following Formula 3:

$$P = \sum a_i x_i \quad \text{Formula 3}$$

P represents the exercise impact factor, $x_i$ represents exercise data of an exercise type, and $a_i$ represents a weight of the exercise type.

**S305:** Obtain an emotion and stress factor of the meal of the user.

Similar to the exercise of the user, an emotion and stress change of the user also affects the heart rate change. Therefore, to avoid that the emotion and stress of the user affect identification of the meal of the user, before it is determined whether the user starts the meal, impact of the emotion and stress of the user on identification of the meal is first eliminated.

The emotion and stress factor of the user may include but is not limited to a stress and emotion variation ΔR of the user. For example, the stress and emotion variation ΔR of the user may be measured by using a sensor (for example, an electrodermal activity sensor).

**S306:** Determine whether the heart rate difference is still greater than or equal to a threshold 2 after impact of the exercise impact factor and the emotion and stress factor is eliminated.

For example, whether the user starts the meal may be determined by using the following Formula 4:

$$\Delta HR - \alpha P - \beta \Delta R \geq TH2 \quad \text{Formula 4}$$

Herein, ΔHR represents the heart rate difference, P represents the exercise impact factor, $\alpha$ represents a weight of the exercise impact factor, ΔR represents the emotion and stress factor, $\beta$ represents a weight of the emotion and stress factor, and TH2 represents the threshold 2.

It can be learned that a change of the current heart rate of the user relative to the resting heart rate may be caused by impact of exercise, emotion and stress, and the meal of the user. Impact of the exercise, and the emotion and stress of the user on the heart rate change of the user may be eliminated by using Formula 4. In this case, after the impact of the exercise, and the emotion and stress of the user is eliminated, if the heart rate change of the user still exceeds a specific value, it indicates that the user is currently having the meal; otherwise, the user does not have the meal.

In other words, when the heart rate difference is still greater than or equal to the threshold 2 after the impact of the exercise impact factor and the emotion and stress factor is eliminated, step S307 is performed, that is, it is determined that the user starts the meal; otherwise, step S308 is performed, that is, it is determined that the user does not have the meal.

**S307:** Determine that the user starts the meal.

If it is determined that the user currently starts the meal, the data collected by the sensor after the current time point and before the user ends the meal is data during the meal of the user.

**S308:** Determine that the user does not have the meal.

If it is determined that the user does not have the meal, steps S301 to S308 may be repeatedly performed, that is, whether the user starts the meal is determined in real time.

It can be learned from steps S301 to S308 that, because the heart rate of the user changes due to the meal, in a process of obtaining the heart rate value of the user in real time, a time point at which the user starts the meal may also be identified based on the heart rate of the user.

Correspondingly, identifying that the meal ends may include but is not limited to the following two manners:

(1) The user manually confirms that the meal ends.

In this case, the user may actively enter an operation of ending the meal to the electronic device (for example, the electronic device 100), so that the electronic device identifies that the user ends the meal, and ends obtaining the meal-related data.

(2) Determine, based on the collected data, whether to end the meal.

When the user ends the meal, factors such as a cardiovascular feature and an exercise mode of the user may be changed. Therefore, whether to end the meal may be determined by collecting data used to reflect the factors such as the cardiovascular feature and the exercise mode of the user.

A manner of determining, based on different collected data, that the meal ends may include but is not limited to the following manners:

(a) Determine, based on hand exercise data of the user, whether the user ends the meal.

The hand exercise data of the user may be obtained by using a wearable device (for example, the electronic device 300), and the user uses a hand wearing the wearable device for the meal.

The hand exercise data of the user may include but is not limited to one or more of the following: a hand exercise range, a hand exercise speed, a hand exercise frequency, and the like.

For example, when the hand exercise range of the user is less than a threshold and the exercise speed decreases to be less than a threshold, it is determined that the user ends the meal.

(b) Determine, based on physical exercise data of the user, whether the user ends the meal.

The physical exercise data of the user may be obtained by using the wearable device (for example, the electronic device 300).

The physical exercise data of the user may include but is not limited to one or more of the following: exercise time, an exercise distance, an exercise pace, an exercise frequency, an exercise speed, and the like. The physical exercise data indicates an exercise mode of the user, and the exercise mode includes but is not limited to sitting, walking, running, jumping, and the like.

For example, when the physical exercise data of the user reflects that the exercise mode of the user is changed from sitting to walking, and exercise time of walking exceeds a threshold, it is determined that the user ends the meal.

(c) Determine, based on data related to the cardiovascular feature of the user, whether the user ends the meal.

The data related to the cardiovascular feature may include but is not limited to one or more of the following: the heart rate, a ventricular beat interval, blood oxygen saturation, and the like.

For example, when the related data reflects that the cardiovascular feature of the user slows down, it is determined that the user ends the meal.

It may be understood that, the collected data is not limited to the data mentioned above, and the manner of identifying that the user ends the meal is not limited to the content mentioned above. This is not limited in embodiments of this application.

**S202:** Calculate a meal volume and a meal speed of the user based on the meal-related data collected by the sensor.

After the time points at which the user starts and ends the meal are identified, the meal volume and the meal speed of the user during the meal may be calculated based on the meal-related data of the user during the meal.

Specifically, the meal-related data of the user during the meal may be input into a meal model, to obtain, through identification, the meal volume and the meal speed of the user during the meal. The meal model is obtained through training based on meal-related data during a meal with a known meal volume and meal speed.

For example, the meal volume and the meal speed may be calculated by using the following Formula 5:

$$Q_{meal}, V_{meal} = F(\Delta HR, \Delta R, f_{hand}, n_{hand}, r_{hand} K) \quad \text{Formula 5}$$

Herein, $Q_{meal}$ represents the meal volume, $V_{meal}$ represents the meal speed, $\Delta HR$ represents a heart rate variation, $\Delta R$ represents an electrodermal activity variation, $f_{hand}$ represents the hand exercise frequency, $n_{hand}$ represents a quantity of hand exercise times, $r_{hand}$ represents the hand exercise range, and $F(\cdot)$ represents the meal model, where an input of the meal model includes $\Delta HR$, $\Delta R$, $f_{hand}$, $n_{hand}$, $r_{hand}$, and an output includes $Q_{meal}$, $V_{meal}$. For example, the meal model may be obtained by training, by using a multivariate linear regression algorithm, a large amount of meal-related data during the meal with the known meal volume and meal speed.

It can be learned from Formula 5 that the meal-related data may include a heart rate value, an electrodermal activity value, a hand exercise frequency, a quantity of hand exercise times, a hand exercise range, and the like that are collected by the sensor from a moment at which the user starts the meal to a moment at which the user ends the meal. The meal volume and the meal speed can be obtained through calculation based on user-related data and by using the meal model. The heart rate value may be collected by using the bone conduction sensor, the electrodermal activity value may be collected by using the electrodermal activity sensor, and the hand exercise frequency, the quantity of hand exercise times, and the hand exercise range may be collected by using an inertial sensor like the gyroscope sensor or the acceleration sensor.

**S203:** Obtain the actual blood glucose curve of the user under the meal volume and the meal speed.

The actual blood glucose curve may be formed by connecting a plurality of blood glucose values, and the plurality of blood glucose values are blood glucose values collected by the electronic device (for example, the electronic device 200) in a process from starting to ending the meal of the user. The actual blood glucose curve indicates a blood glucose change of the user during the meal.

For example, it is assumed that the user spends 30 minutes in total from starting the meal to ending the meal. In this case, the actual blood glucose curve includes a plurality of blood glucose values collected within the 30 minutes.

**S204:** Obtain blood glucose curves of a plurality of members having a same meal volume and meal speed in healthy people.

In the blood glucose management method, a diet database may be managed and maintained, and the diet database may store a plurality of blood glucose values during a meal of the healthy people, that is, people who do not have diabetes. In other words, blood glucose curves of members having different meal volumes and different meal speeds in the healthy people may be obtained by using the diet database.

During specific implementation, blood glucose curves of a plurality of members that are in the healthy people and that have a same meal volume and meal speed as the user may be obtained from the diet database based on the meal volume and the meal speed of the user.

**S205:** Determine the standard blood glucose curve of the healthy people based on the plurality of blood glucose curves.

Due to body differences of different members in the healthy people, blood glucose curves of the different members are different even if these members do not have diabetes. The difference may be reflected as different blood glucose values at a single time point and different duration (different meal duration). Therefore, the plurality of blood glucose curves need to be adjusted, by using a blood glucose normalization method, to represent the standard blood glucose curve of the healthy people during the meal with the same meal volume and meal speed. The standard blood glucose curve indicates a blood glucose change of the healthy people during the meal with the same meal volume and meal speed of the user.

For example, the blood glucose normalization method may include the following two steps.

**Step 1:** Perform stretching or compression transformation on the plurality of blood glucose curves in time domain, and/or perform stretching or compression transformation on the plurality of blood glucose curves in amplitude, so that average values of the plurality of blood glucose curves in time domain and amplitude are equal.

When stretching or compression transformation are performed on the plurality of blood glucose curves in time domain, the plurality of blood glucose curves may be transformed by using an average value of duration of the plurality of blood glucose curves in time domain as a reference, so that a plurality of transformed blood glucose curves have equal duration in time domain.

For example, it is assumed that there are two blood glucose curves, one blood glucose curve describes a blood glucose change within 30 minutes, in other words, duration of the blood glucose curve in time domain is 30 minutes, and the other blood glucose curve describes a blood glucose change within 20 minutes, in other words, duration of the blood glucose curve in time domain is 20 minutes. In this case, an average value of duration of the two blood glucose curves in time domain is (30+25)/2=25 minutes. Therefore, when the blood glucose curves are transformed, a previous blood glucose curve needs to be compressed from 30 minutes to 25 minutes in time domain, and a next blood glucose curve needs to be stretched from 30 minutes to 25 minutes in time domain.

In addition, when stretching or compression transformation are performed on the plurality of blood glucose curves in amplitude, the plurality of blood glucose curves may be transformed by using an average value of blood glucose values of the plurality of blood glucose curves in amplitude as a reference, so that a plurality of transformed blood glucose curves have equal average blood glucose values in amplitude.

For example, it is assumed that there are two blood glucose curves, an average value of a plurality of blood glucose values included on one blood glucose curve is 6.2 mmol/L, and an average value of a plurality of blood glucose values included on the other blood glucose curve is 6.8 mmol/L. In this case, an average blood glucose value of the two blood glucose curves in amplitude is (6.2+6.8)/2=6.5 mmol/L. Therefore, when the blood glucose curves are transformed, a previous blood glucose curve needs to be stretched in amplitude, so that an average blood glucose value in amplitude is adjusted from 6.2 mmol/L to 6.5 mmol/L, and a next blood glucose curve needs to be compressed in amplitude, so that an average blood glucose value in amplitude is adjusted from 6.8 mmol/L to 6.5 mmol/L.

It may be understood that, if a blood glucose curve satisfies a time domain requirement before transformation, the blood glucose curve does not need to be stretched or compressed in time domain. Similarly, if the blood glucose curve satisfies an amplitude requirement before transformation, the blood glucose curve does not need to be stretched or compressed in amplitude.

**Step 2:** Perform point-by-point average value calculation on the plurality of transformed blood glucose curves, and determine a curve formed by connecting the average values as the standard blood glucose curve.

In other words, a blood glucose value at any time point on the standard blood glucose curve is an average value of blood glucose values at the time point on the plurality of transformed blood glucose curves.

For ease of understanding, the following describes the blood glucose normalization method by using a specific example.

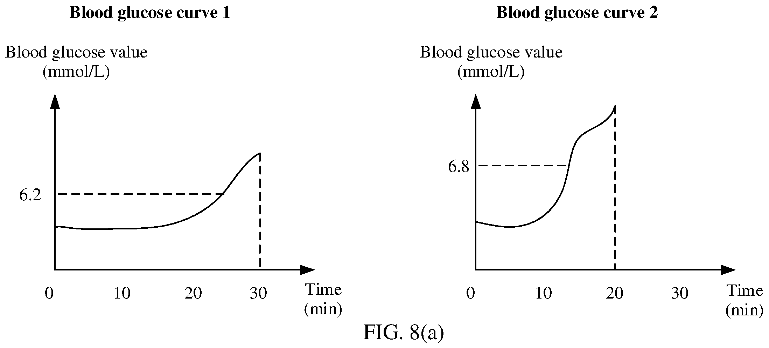

FIG. 8(a) to FIG. 8(d) are a diagram of an example of a process in which two blood glucose curves are normalized into a standard blood glucose curve.

FIG. 8(a) shows an example of a blood glucose curve 1 and a blood glucose curve 2 of members that are in the healthy people and that have the same meal volume and meal speed as the user. The blood glucose curve 1 and the blood glucose curve 2 describe a blood glucose change with time during a meal of the members. It can be learned from the blood glucose curve 1 that the member spends 30 minutes on the meal, and an average blood glucose value is 6.2 mmol/L. It can be learned from the blood glucose curve 2 that the member spends 20 minutes on the meal, and an average blood glucose value is 6.8 mmol/L.

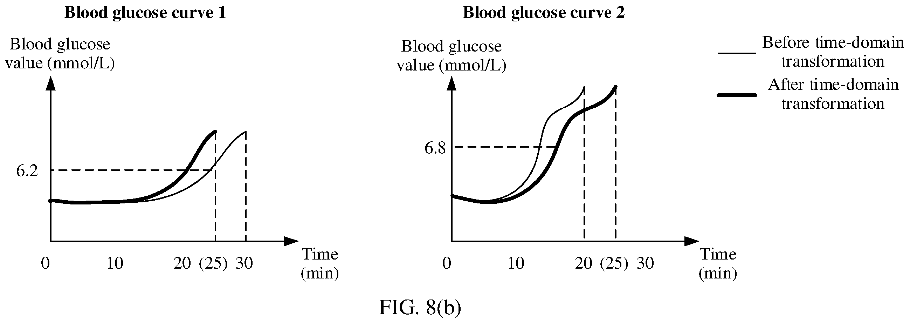

FIG. 8(b) shows an example of a comparison diagram (left) of curves before and after time-domain (horizontal) compression transformation is performed on the blood glucose curve 1, and a comparison diagram (right) of curves before and after time-domain (horizontal) stretching transformation is performed on the blood glucose curve 2. In this way, time lengths of a blood glucose curve 1 and a blood glucose curve 1 that are obtained after time-domain transformation are both 20 minutes in time domain.

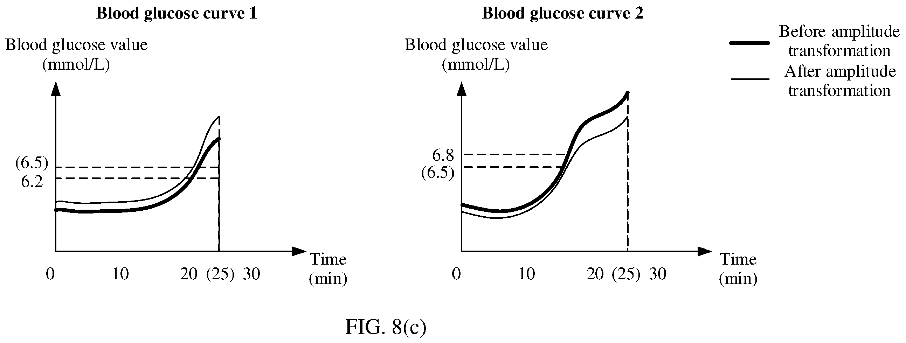

On the basis of FIG. 8(b), FIG. 8(c) shows an example of a comparison diagram (left) of curves before and after amplitude (vertical) stretching transformation is performed on the blood glucose curve 1 obtained after the time-domain transformation, and a comparison diagram (right) of curves before and after amplitude (vertical) compression transformation is performed on the blood glucose curve 2 obtained after the time-domain transformation. In this way, average blood glucose values of a blood glucose curve 1 and a blood glucose curve 2 that are obtained after amplitude transformation are both 6.5 mmol/L in amplitude.

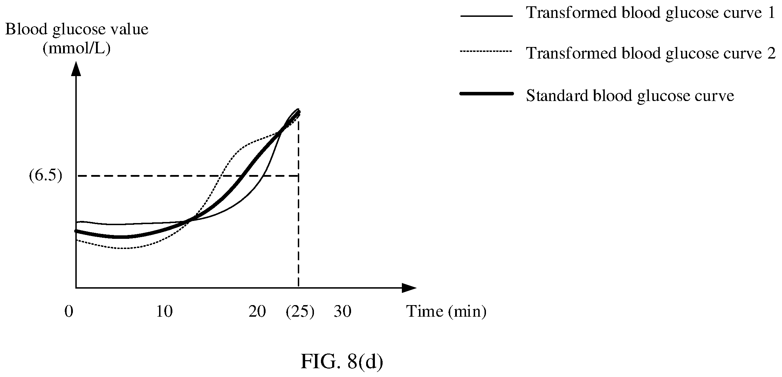

FIG. 8(d) shows an example of a transformed blood glucose curve 1, a transformed blood glucose curve 2, and a standard blood glucose curve on a same coordinate axis. The transformed blood glucose curve 1 and the transformed blood glucose curve 2 are blood glucose curves obtained after time-domain and amplitude transformation shown in FIG. 8(c), and the standard blood glucose curve is obtained based on the transformed blood glucose curve 1 and the transformed blood glucose curve 2. A blood glucose value at a time point of the standard blood glucose curve is an average value of blood glucose values of the transformed blood glucose curve 1 and the transformed blood glucose curve 2 at the same time point.

It may be understood that FIG. 8(a) to FIG. 8(d) show only an example of a process of determining the standard blood glucose curve based on two blood glucose curves. In this embodiment of this application, the standard blood glucose curve may be obtained based on 4 or 10 blood glucose curves. This is not limited in embodiments of this application.

**S206:** Evaluate diet health of the user based on the actual blood glucose curve and the standard blood glucose curve.

Specifically, the diet health of the user may be evaluated by comparing a difference between the actual blood glucose curve and the standard blood glucose curve. A larger difference between the actual blood glucose curve and the standard blood glucose curve indicates an unhealthier diet of the user, and a smaller difference between the actual blood glucose curve and the standard blood glucose curve indicates a healthier diet of the user.

For example, a feature of the curve may be extracted to quantize the feature of the curve, and then the difference between the actual blood glucose curve and the standard blood glucose curve is compared by using the feature.

The feature of the curve may include but is not limited to one or more of the following:

(1) Maximum value Kmax of a single blood glucose change

The maximum value Kmax of the blood glucose change may be a maximum slope on the curve. For example, the slope K may be determined by using the following Formula 6:

$$K = \frac{\Delta y}{\Delta x} = \frac{y_2 - y_1}{x_2 - x_1} \quad \text{Formula 6}$$

Herein, $\Delta x$ represents a time variation, $\Delta y$ represents a blood glucose variation, and $(x_1, y_1)$ and $(x_1, y_1)$ are two coordinate points on the curve. $(x_1, y_1)$ indicates that a blood glucose value on the curve is $y_1$ at a time point $x_1$, and $(x_2, y_2)$ indicates that a blood glucose value on the curve is $y_2$ at a time point $x_2$.

(2) Non-stationary blood glucose time $T_N$

The non-stationary blood glucose time $T_N$ may be determined by using the following Formula 7:

$$T_N = T_{ALL} - T_S \quad \text{Formula 7}$$

Herein, $T_{ALL}$ represents total duration of the curve in time domain, and $T_S$ represents stationary blood glucose time on the curve. For example, a stationary blood glucose moment on the curve may be determined by using a blood glucose fluctuation coefficient, to determine the stationary blood glucose time.

(3) Quantities of blood glucose values in different blood glucose change rate categories

In other words, blood glucose values included on the curve may be segmented based on a blood glucose change rate, and quantities of blood glucose values in different blood glucose change rate categories are counted.

For example, the blood glucose change rate c may be determined by using the following Formula 8:

$$c = \frac{std}{\sqrt{avg}}, c \leq 0.07 \quad \text{Formula 8}$$

Herein, std represents a standard deviation of the blood glucose values included on the curve, and avg represents an average value of the blood glucose values included on the curve.

(4) Quantity F of blood glucose fluctuation times

The quantity F of blood glucose fluctuation times may be determined according to the following Formula 9:

$$F = F_{up} + F_{down} \quad \text{Formula 9}$$

$F_{up}$ represents a quantity of times that blood glucose rises on the curve, and $F_{down}$ represents a quantity of times that blood glucose drops on the curve.

(5) Time x and amplitude y of the single blood glucose change

Herein, (x, y) is a point on the curve, and represents a blood glucose value y on the curve at a time point x.

(6) Curve envelope

It may be understood that the curve may further include another feature used to represent a feature of the curve. This is not limited in embodiments of this application.

After curve features of the actual blood glucose curve and the standard blood glucose curve are extracted, a difference Diff between the two curves may be calculated by using the following Formula 10:

$$\text{Diff} = \sum_{i}^{n} (q_1 - q_0)_i \cdot \lambda_i \quad \text{Formula 10}$$

Herein, $(q_1 - q_0)_i$ represents a difference between the actual blood glucose curve and the standard blood glucose curve for a same curve feature, $q_1$ represents the curve feature in the actual blood glucose curve, and $q_0$ represents the curve feature in the standard blood glucose curve. The difference indicates the diet health of the user. A larger difference indicates an unhealthier diet of the user, and a smaller difference indicates a healthier diet of the user. For example, the curve feature may include the maximum value Kmax of the single blood glucose change, the non-stationary blood glucose time $T_N$, the quantities of blood glucose values in different blood glucose change rate categories, the quantity of blood glucose fluctuation times F, the time x and the amplitude y of the single blood glucose change, the curve envelope, and the like. n represents a quantity of curve features, and $\lambda_i$ represents a weight of the curve features.

It can be learned that the difference between the actual blood glucose curve and the standard blood glucose curve of a single diet of the user can be calculated by using Formula 10. A larger value of Diff indicates a larger difference between the actual blood glucose curve and the standard blood glucose curve of the diet of the user and a worse blood glucose health status of the diet of the user, and a smaller value of Diff indicates a smaller difference between the actual blood glucose curve and the standard blood glucose curve of the diet of the user and a better blood glucose health status of the diet of the user.

Further, for a plurality of diets of the user, a difference $Diff_i$ between an actual blood glucose curve and a standard blood glucose curve of each diet of the user may be calculated by using the foregoing method, and a blood glucose status of the plurality of diets of the user within a period of time may be evaluated by using the following Formula 11:

$$S = \sum \alpha_i \beta(t) Diff_i \quad \text{Formula 11}$$

Herein, S represents an overall health score of the plurality of diets of the user within the period of time, $\alpha_i$ represents a weight of a diet i within the period of time, $\beta(t)$ is a time attenuation factor, where the time attenuation factor implements a small weight of a diet obtained through early statistics collection, and $Diff_i$ represents a difference between an actual blood glucose curve and a standard blood glucose curve of the diet i of the user within the period of time.

It can be learned that, a larger value of S indicates a larger overall difference between the actual blood glucose curve and the standard blood glucose curve of the plurality of diets of the user within the period of time and a worse diet health status of the user within the period of time, and a smaller value of S indicates a smaller overall difference between the actual blood glucose curve and the standard blood glucose curve of the plurality of diets of the user within the period of time and a better diet health status of the user within the period of time.

In some embodiments, the actual blood glucose curve and the standard blood glucose curve may be further displayed. For example, the actual blood glucose curve and the standard blood glucose curve may be displayed on a same coordinate axis. In this way, the user can directly learn of a health difference between the user and the healthy people by comparing a difference between the curves.

In general, according to the blood glucose management method provided in Embodiment 2, a difference between blood glucose of the user and blood glucose of the healthy people can be quantified by using the blood glucose value of the user and in a unit of a single diet of the user, to evaluate blood glucose health of the user after the meal, and help the user better learn of a physical condition of the user.

**Embodiment (3)**

In daily life, exercise also affects a change in a blood glucose concentration of a user. For a patient having diabetes, because the patient lacks an effective ability to adjust blood glucose, inappropriate exercise may aggravate the patient's condition and endanger the patient's health.

This embodiment of this application provides a blood glucose management method, to comprehensively evaluate an exercise risk of the user based on a plurality of reference factors including blood glucose, a heart rate, a breath, and the like before, during, and after exercise of the user. This helps the user exercise in a safer and healthier way, and ensures safe and healthy exercise for the user.

Specifically, in the blood glucose management method, an exercise risk score of the user may be determined based on an exercise reference factor of the user before the exercise, and a recommendation for the exercise of the user is output before the exercise of the user. In the blood glucose management method, whether the user has a hyperglycemia or hypoglycemia risk during the exercise or after the exercise may be further evaluated based on an exercise reference factor of the user during the exercise, and an exercise alarm is output during the exercise or after the exercise of the user. In the blood glucose management method, an exercise process of the user may be further analyzed based on an exercise reference factor of the user after the exercise, and an exercise evaluation report is output. The exercise reference factor includes blood glucose and a parameter used to reflect a vital sign of the user, for example, a heart rate, a breath, or blood pressure.

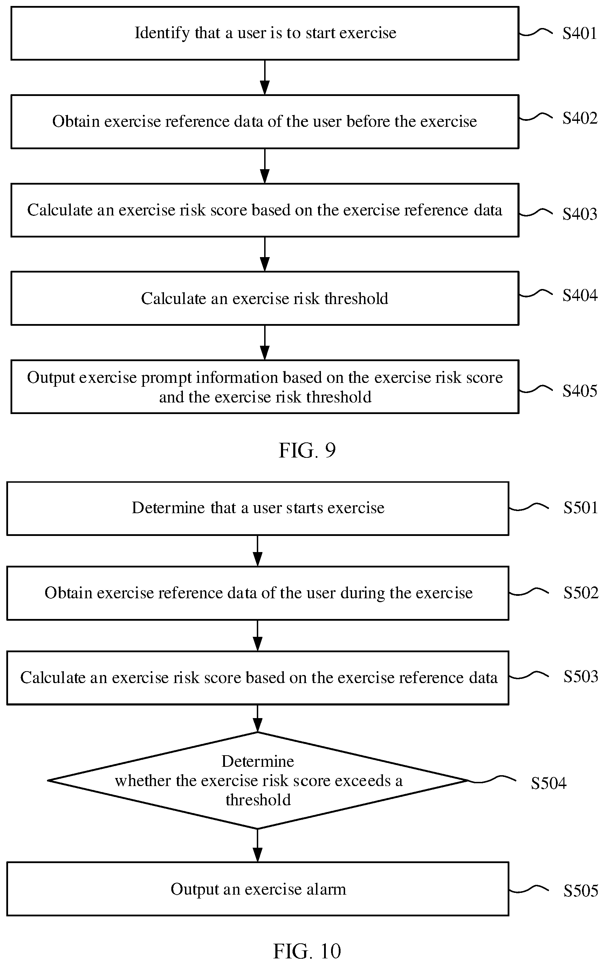

**The following describes, with reference to FIG. 9, a detailed process of evaluating the exercise risk of the user before the exercise.**

FIG. 9 is a schematic flowchart of evaluating the exercise risk of the user before the exercise according to this embodiment of this application.

As shown in FIG. 9, evaluating the exercise risk of the user before the exercise mainly includes the following steps.

**S401:** Identify that the user is to start the exercise.

For example, it may be identified, in the following two manners, that the user is to start the exercise.

(1) Detect an operation entered by the user for indicating that the exercise is to start.
For example, the electronic device 100 may detect an operation entered by the user on the electronic device 100 for indicating that the exercise is to start. In response to the operation, the electronic device 100 identifies that the user is to start the exercise.
(2) Detect that current time reaches exercise time preset by the user.

In this case, the exercise time preset by the user needs to be obtained in advance. For example, the user presets to perform rope jumping exercise at 5:00 p.m., and when the time reaches 5:00 p.m., it is determined that the user is to start the exercise. Alternatively, the exercise time of the user is determined in advance based on an exercise habit of the user. For example, it is identified that the user performs running exercise at 9:00 a.m. every day, and when the time reaches 9:00 a.m., it may be predicted that the user is to start the exercise.

It may be understood that a manner of identifying that the user is to start the exercise is not limited in embodiments of this application.

**S402:** Obtain exercise reference data of the user before the exercise.

Exercise reference data of the user within preset duration before the exercise may be obtained. For example, the preset duration may be 20 minutes, 30 minutes, or the like. This is not limited in embodiments of this application.

The exercise reference data is used to predict an exercise risk of the user. The exercise reference data may include but is not limited to blood glucose data, a heart rate, blood pressure, a breath, and the like. The blood glucose data may include but is not limited to one or more of the following: a blood glucose value, a quantity of blood glucose fluctuation times, a blood glucose fluctuation amplitude, and the like.

**S403:** Calculate an exercise risk score based on the exercise reference data.

The exercise risk score is used to predict a risk of the exercise that the user is to start. For example, a higher exercise risk score indicates a higher risk of the exercise that the user is to start, and a lower exercise risk score indicates a lower risk of the exercise that the user is to start.

For example, the exercise risk score G may be calculated by using the following Formula 12:

$$G = \sum a_i\, x_i \quad \text{Formula 12}$$

Herein, $x_i$ represents a level of an $i^{th}$ piece of exercise reference data, and $a_i$ represents a weight of the $i^{th}$ piece of exercise reference data.

A level of the exercise reference data may be determined based on a value of the exercise reference data. For example, the level of the exercise reference data may be determined based on a mapping table between exercise reference data and levels, and the mapping table includes levels corresponding to exercise reference data of different values. For example, the exercise reference data includes blood pressure, a heart rate, and blood glucose. Table 1 shows an example of a mapping table between exercise reference data and levels.

Table 1

| Exercise reference data | Value range | Level |
|---|---|---|
| Blood pressure | Systolic blood pressure≥180<br>Diastolic blood pressure≥110 | 3 |
| | 179≥systolic blood pressure≥160<br>109≥diastolic blood pressure≥100 | 2 |
| | 159≥systolic blood pressure≥140<br>99≥diastolic blood pressure≥90 | 1 |
| | Systolic blood pressure≤139<br>Diastolic blood pressure≤89 | 0 |
| Heart rate | Maximum heart rate percentage≥90% | 5 |
| | 90%>maximum heart rate percentage≥80% | 4 |
| | 80%>maximum heart rate percentage≥70% | 3 |
| | 70%>maximum heart rate percentage≥60% | 2 |
| | 60%>maximum heart rate percentage≥50% | 1 |
| | Maximum heart rate percentage<50% | 0 |
| Blood glucose | Fasting blood glucose>11.1 or fasting blood glucose<2.9 | 2 |
| | 11.1≥fasting blood glucose>6.1 or 3.9>fasting blood glucose≥2.9 | 1 |
| | 6.1≥fasting blood glucose≥3.9 | 0 |

It can be learned from Table 1 that the exercise reference data corresponds to different levels in different value ranges. A larger deviation of a value from a normal range of the exercise reference data indicates a higher level of the exercise reference data and a higher risk of the exercise that the user is to start.

Specifically, when the exercise risk score is calculated, an average value of data in obtained exercise reference data within a period of time before the exercise may be first calculated, then, a level of the data in the exercise reference data is determined by using a mapping table between values and levels similar to that shown in Table 1, and finally, the exercise risk score is calculated by using the foregoing Formula 12.

In some implementations, when the exercise of the user includes a plurality of exercise items, the exercise risk score may be further determined based on exercise information that is of the plurality of exercise items and that is entered by the user. The exercise information may include but is not limited to one or more of the following: an exercise type, an exercise intensity, exercise duration, and the like.

For example, the exercise risk score may be determined according to the following Formula 13:

$$G = \sum a_i\, x_i \cdot \sum b_j\, y_j \quad \text{Formula 13}$$

Herein, $x_i$ represents a level of an $i^{\text{th}}$ piece of exercise reference data, $a_i$ represents a weight of the $i^{\text{th}}$ piece of exercise reference data, $y_j$ represents exercise information of a $j^{\text{th}}$ exercise item, and $b_j$ represents a weight of the $j^{\text{th}}$ exercise item.

**S404:** Calculate an exercise risk threshold.

The exercise risk threshold may be determined based on metrics such as basic information of the user and a blood glucose feature that reflects a blood glucose status of the user within a period of time.

The basic information of the user may include but is not limited to one or more of the following: an age, a gender, a body mass index (Body Mass Index, BMI), a disease type, information indicating whether the user has a complication like hypertension/hyperlipidemia, and the like. The body mass index is used to measure body fatness and thinness, and a health status.

The blood glucose feature may include but is not limited to one or more of the following: an average blood glucose value within a period of time, an average blood glucose change rate, and a variation coefficient. The variation coefficient is a percentage of a standard blood glucose deviation and an average blood glucose value. The variation coefficient is used to reflect a fluctuation status of blood glucose of the user within the period of time.

For example, the exercise risk threshold TH may be calculated by using the metric of the user according to the following Formula 14:

$$TH = \sum c_k t_k \quad \text{Formula 14}$$

Herein, $t_k$ represents a level of a $k^{\text{th}}$ metric of the user, where the metric level may be determined based on a mapping table between metrics and levels, and $c_k$ represents a weight of the $k^{\text{th}}$ metric.

It should be understood that, the mapping table between metrics and levels includes different value ranges of the metric or different cases of the metric, and levels corresponding to the different value ranges or the different cases. For example, for the mapping table, refer to Table 1. For example, when the body mass index of the user is greater than 30 or less than 15, a level is 3; when the body mass index of the user is from 25 to 30 or from 15 to 20, a level is 2; and when the body mass index of the user is between 20 and 25, a level is 1. For another example, when the gender of the user is male, a level is 2; or when the gender of the user is female, a level is 1.

**S405:** Output exercise prompt information based on the exercise risk score and the exercise risk threshold.

The output exercise prompt information may be determined by comparing the exercise risk score G with the exercise risk threshold TH. The exercise prompt information indicates a recommendation for the exercise of the user.

For example, the following lists exercise prompt information in several different cases.

(1) When $G < \lambda \cdot TH$ ($\lambda < 1$), the output exercise prompt information is that exercise is recommended.
When the exercise risk score G is less than $\lambda$ times of the exercise risk threshold, it indicates that the exercise risk is low or there is no risk currently, and the user is recommended to exercise.
(2) When $TH > G > \lambda \cdot TH$, the output exercise prompt information is an exercise precaution or an optimized exercise scheme.

When the exercise risk score G is greater than $\lambda$ times of the exercise risk threshold and less than the exercise risk threshold, it indicates that there may be an exercise risk currently. Therefore, the user may be reminded of a related exercise precaution, or an exercise scheme of the user may be optimized, to reduce the exercise risk of the user as much as possible.

The exercise precaution may include: reminding the user to perform warm-up exercise before the exercise, reminding the user to pay attention to a heart rate change, drink water, and perform cool-down exercise after the exercise, and reminding, based on a blood glucose level of the user within preset duration before the exercise, the user to take medicine in advance when there is a risk of hyperglycemia, or supplement sugar in advance when there is a risk of hypoglycemia.

Optimizing the exercise scheme of the user is mainly used in a case in which the user enters exercise information of a plurality of exercise items. In this case, the exercise risk score is determined based on the exercise reference data and the exercise information of the exercise items. For details, refer to Formula 13. Optimizing the exercise scheme may include: reducing an exercise intensity and/or exercise duration of one or more exercise items entered by the user. The adjusted exercise item may be an exercise item that contributes more to the exercise risk score.

For example, exercise to be started by the user includes rope jumping for 30 minutes and running for 30 minutes. In a calculated exercise risk score, a product of exercise information and a weight of rope jumping is greater than a product of exercise information and a weight of running. Therefore, rope jumping contributes more to the exercise risk score. In this way, when the exercise scheme is optimized, exercise time of rope jumping may be reduced, and the exercise time of rope jumping is shortened to 10 minutes. In this way, the output exercise prompt information may include: "To ensure your safety, it is advised to shorten rope jumping time to 10 minutes".

(3) When G>TH, the output exercise prompt information is that exercise is not recommended.

When the exercise risk score is greater than the exercise risk threshold, it indicates that the exercise risk is high currently, and the user is not recommended to exercise.

It can be learned from steps S401 to S405 that, before the user exercises, a physical condition of the user before the exercise may be comprehensively considered to evaluate whether the user is currently suitable for exercise, so as to ensure that the user can exercise as healthily and safely as possible.

**The following describes, with reference to FIG. 10, a detailed process of evaluating the exercise risk of the user during the exercise.**

FIG. 10 is a schematic flowchart of evaluating the exercise risk of the user during exercise according to this embodiment of this application.

As shown in FIG. 10, evaluating the exercise risk of the user during the exercise mainly includes the following steps.

**S501:** Determine that the user starts exercise.

For example, exercise data of the user may be collected by using a sensor. When the exercise data indicates that the user changes from a non-exercise state to an exercise state, it is determined that the user starts exercise.

**S502:** Obtain exercise reference data of the user during the exercise.

The exercise reference data is used to represent an exercise status of the user. The exercise reference data may include but is not limited to one or more of the following information: blood glucose, a heart rate, blood pressure, a breath, and the like.

For example, the electronic device 200 may collect blood glucose of the user, and the electronic device 300 may collect a heart rate, blood pressure, and a breath of the user.

**S503:** Calculate an exercise risk score based on the exercise reference data.

The exercise risk score indicates an exercise risk during the exercise. For example, the exercise risk score may be determined according to the following Formula 15:

$$G = \gamma(t) \sum c_i z_i \quad \text{Formula 15}$$

Herein, $z_i$ represents a level of an $i^{th}$ piece of exercise reference data, $c_i$ represents a weight of the $i^{th}$ piece of exercise reference data, and $\gamma(t)$ represents a time attenuation factor, where the time attenuation factor is a function that monotonically increases with time t, and the time attenuation factor is used to simulate a delayed hyperglycemia/hypoglycemia risk that gradually increases with time.

A level of the exercise reference data may be determined based on a value of the exercise reference data. For example, the level of the exercise reference data may be determined based on a mapping table between exercise reference data and levels, and the mapping table may include levels corresponding to exercise reference data of different values.

It can be learned that a higher exercise risk score indicates a higher exercise risk, and a lower exercise risk score indicates a lower exercise risk.

In some implementations, the exercise risk score may alternatively be determined based on basic information of the user. The basic information may include information such as a height, a weight, and a medical history of the user. A manner of calculating the exercise risk score is not limited in embodiments of this application.

**S504:** Determine whether the exercise risk score exceeds a threshold.

The exercise risk score is a parameter obtained through real-time calculation based on the exercise reference data obtained by the user during the exercise. Therefore, the exercise risk score may change with time.

When the exercise risk score exceeds the threshold, it indicates that there is an exercise risk in the exercise performed by the user, and step S505 is performed; otherwise, whether the exercise risk score exceeds the threshold continues to be determined.

In this embodiment of this application, the threshold may alternatively be referred to as fourth threshold.

**S505:** Output an exercise alarm.

The exercise alarm may include a sound prompt, a vibration prompt, a text prompt, or the like. The exercise alarm is used to: remind the user to terminate the current exercise, and remind the user to supplement food, take medicine, perform cool-down exercise, and the like in time.

In addition, optionally, after preset duration (for example, fourth duration) after the user ends the exercise, the exercise alarm may be further output again, to remind the user to supplement food, take medicine, and the like in time, to avoid a hyperglycemia phenomenon caused by the exercise and prevent the user from having a delayed hypoglycemia symptom.

It can be learned from steps S501 to S505 that, during the exercise of the user, the exercise status of the user may be monitored in real time, a plurality of pieces of vital sign data of the user during the exercise are comprehensively considered, the exercise risk of the user is evaluated, and when the exercise risk is high, the user is reminded in time to interrupt the exercise, to ensure health and safety of the user during the exercise.

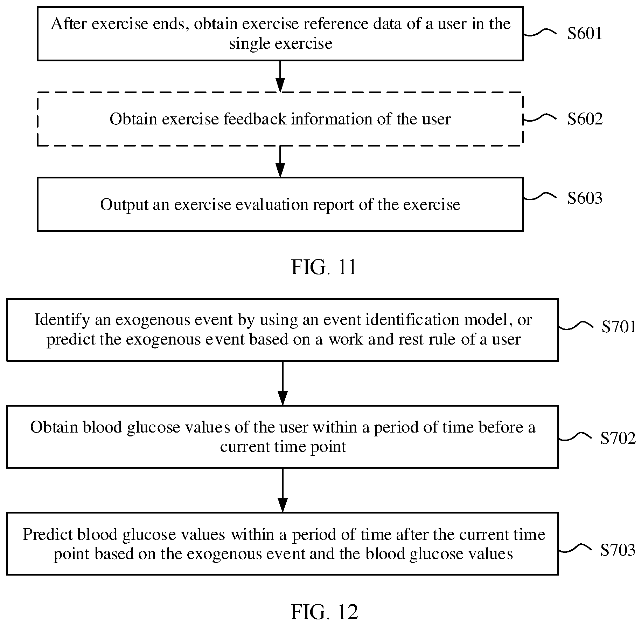

**The following describes, with reference to FIG. 11, a detailed process of evaluating the exercise risk of the user after the exercise.**

FIG. 11 is a schematic flowchart of evaluating the exercise risk of the user after exercise according to this embodiment of this application.

As shown in FIG. 11, evaluating the exercise risk of the user after the exercise may include the following steps.

**S601:** After the exercise ends, obtain exercise reference data of the user in the single exercise.

The exercise reference data may include data such as blood glucose, a heart rate, blood pressure, a breath, exercise time, and an exercise distance during the single exercise of the user. The blood glucose data may include a maximum value or a minimum value in a plurality of blood glucose values collected during the single exercise of the user, a blood glucose fluctuation amplitude, a quantity of blood glucose fluctuation times, a quantity of times that a hyperglycemia/hypoglycemia threshold is exceeded, and the like.

It should be understood that the exercise reference data is not limited in embodiments of this application.

**S602:** Obtain exercise feedback information of the user.

The exercise feedback information may include exercise feelings fed back by the user after the exercise ends. For example, the exercise feedback information may include a score of the user on an exercise intensity, a feedback on body inappropriateness, and the like.

It should be noted that step S602 is an optional step.

**S603:** Output an exercise evaluation report of the exercise.

The exercise evaluation report may be determined based on the exercise reference data of the user, and the exercise evaluation report may be used to evaluate impact of the exercise on the user.

The impact of the exercise on the user may be represented by using an exercise impact score. A higher exercise impact score indicates greater impact of the exercise on the user, and a lower exercise impact score indicates less impact of the exercise on the user.

For example, the exercise impact score may be determined by using the following Formula 16:

$$G = \sum d_i w_i \quad \text{Formula 16}$$

Herein, $w_i$ represents a level of an $i^{th}$ piece of exercise reference data, and $c_i$ represents a weight of the $i^{th}$ piece of exercise reference data.

A level of the exercise reference data may be determined based on a value of the exercise reference data. For example, the level of the exercise reference data may be determined based on a mapping table between exercise reference data and levels, and the mapping table may include levels corresponding to exercise reference data of different values.

Further, the exercise evaluation report may further include a recommendation for next exercise, and the recommendation may be determined based on the exercise feedback information of the user.

The recommendation for the next exercise may include an adjustment recommendation for an exercise type, exercise duration, and an exercise intensity of the next exercise. For example, when the exercise feedback information of the user indicates that the exercise intensity is excessively high, the exercise intensity of the next exercise may be appropriately reduced based on the current exercise intensity; when the exercise feedback information of the user indicates that the exercise duration is excessively short, the exercise duration of the next exercise may be appropriately increased based on the current exercise duration; and when the exercise feedback information of the user indicates that arms of the user are sore after the exercise, an exercise type that avoids excessive arm exercise may be selected as the exercise type of the next exercise.

In some implementations, an exercise evaluation report for exercise of the user within a period of time may be further output based on exercise reference data of the user in a plurality of times of exercise, to reflect an exercise status of the user within the period of time, evaluate an exercise risk of the user within the period of time, and help the user learn of a physical condition of the user more comprehensively.

It can be learned from steps S601 to S603 that, when the user ends the exercise, a plurality of pieces of vital sign data of the user during the exercise are comprehensively considered, an exercise status of the user is summarized, and the exercise is used as a reference, to provide a recommendation for next exercise of the user and help the user perform long-term healthy exercise.

**Embodiment (4)**

Diabetes disables a patient's body to normally control a blood glucose concentration. High blood glucose easily causes disordered metabolism in the patient, and low blood glucose easily causes damage to the central nerve of the patient. Therefore, it is vital for the diabetic patient to pay attention to a blood glucose status of the patient in real time. The patient can take medicine or eat food in time based on a blood glucose change of the patient, and exercise appropriately, to control the blood glucose concentration to fluctuate within a normal range, and reduce harm caused by excessively high or low blood glucose.

Further, it is assumed that if a future blood glucose change trend can be learned in advance, it is more effective for a user to control blood glucose of the user. The user may take a corresponding measure in time before the blood glucose is excessively high or low. In this way, a blood glucose concentration can be controlled to fluctuate within a normal range, and harm caused by excessively high or low blood glucose can be avoided in time.

To predict a future blood glucose concentration, a blood glucose prediction method is to obtain, through model training based on known historical blood glucose data of the user, a blood glucose prediction model that can predict the future blood glucose concentration. In this way, when the user enters collected blood glucose values to the blood glucose prediction model, the blood glucose prediction model can output a future blood glucose value of the user.

However, in the blood glucose prediction method, although the future blood glucose value of the user can be predicted, a blood glucose value of the user is affected by an exogenous event, for example, an exercise event, a diet event, or a medication event, and it is not accurate to predict future blood glucose only based on a historical blood glucose value of the user.

This embodiment of this application provides a blood glucose management method. In the method, the exogenous event can be identified by using an event identification model, or the exogenous event can be predicted based on a work and rest rule of the user, and blood glucose values within a period of time after a current time point are predicted based on blood glucose values within a period of time before the current time point and the exogenous event, to implement an effect of predicting the future blood glucose value by using the exogenous event and the historical blood glucose value.

It can be learned that, according to the blood glucose management method provided in this embodiment of this application, the future blood glucose value of the user can be predicted based on the blood glucose value of the user and the exogenous event, and external factors that can affect the blood glucose concentration of the user can be considered as much as possible, to improve blood glucose prediction precision and highlight an association between the exogenous event and blood glucose of the user, so that the user can better plan a healthy exogenous event to effectively avoid harm caused by hyperglycemia or hypoglycemia. In addition, the user does not need to manually enter the exogenous event. This facilitates an operation of the user, and avoids a problem of inaccurate model prediction caused by an incorrect input of the user.

**FIG. 12 is a schematic flowchart of another blood glucose management method according to this embodiment of this application.**

As shown in FIG. 12, the blood glucose management method may include the following steps.

**S701:** Identify the exogenous event by using the event identification model, or predict the exogenous event based on the work and rest rule of the user.

The exogenous event includes one or more of the following: the diet event, the medication event, and the exercise event. Occurrence of the exogenous event causes fluctuation of the blood glucose of the user. Therefore, in the blood glucose management method provided in this embodiment of this application, an exogenous event that has occurred before the current time point or an exogenous event that may occur after the current time point is introduced, to predict a future blood glucose value.

In this embodiment of this application, the exogenous event may alternatively be referred to as a first exogenous event.

The exogenous event may be obtained in the following two manners:

(1) The exogenous event that has occurred before the current time point may be obtained through identification by using the event identification model.

The event identification model is obtained through training based on a historical exogenous event of the user and a historical blood glucose value.

Because a blood glucose concentration of the user is related to the exogenous event, a blood glucose concentration change reflects, to an extent, the exogenous event that occurs on the user. Therefore, the event identification model may be trained by using a blood glucose value and an exogenous event that are historically known by the user, to find a rule between the exogenous event and the blood glucose. In this way, the exogenous event can be obtained through identification based on the event identification model and known blood glucose data.

Based on different exogenous events, the event identification model may include an exercise identification model, a medication identification model, and a diet identification model.

The following separately describes manners of generating the exercise identification model, the medication identification model, and the diet identification model.

(a) Exercise identification model

The exercise identification model may be used to identify exercise start time, an exercise volume, and an exercise type of an exercise event.

For example, the exercise identification model may include an exercise start time identification model, an exercise volume identification model, and an exercise type identification model.

The exercise start time identification model may identify the exercise start time based on values of features such as variability, an average value, and a change rate of blood glucose values within a period of time. For example, when the variability and the average value of the blood glucose values of the user within the period of time satisfy a preset condition, it is determined that the exercise start time is specified time that has a preset association with the period of time.

The preset condition may be determined based on a blood glucose feature of historical blood glucose data and exercise start time of a historical exercise event. For example, the preset condition may be a condition that is satisfied by features of blood glucose in most (for example, 80%) of historical exercise events within a period of time before and after exercise. For example, it is learned from the historical exercise event and the historical blood glucose data that blood glucose variability of the user is large within one hour after the user starts exercise. Therefore, if it is identified that blood glucose variability of the user within one hour is large, it is determined that the user has an exercise event, where start time of the one hour is exercise start time of the exercise event.

The exercise volume identification model may be a regression model, for example, a support vector classification (Support Vector Classification, SVC) model, a random forest model, or a linear regression model. The model $\Phi1(\cdot)$ may be represented by using the following Formula 17:

$$L1 = \Phi1(G) \quad \text{Formula 17}$$

G represents consecutive blood glucose values recorded within a period of time, and L1 represents an exercise volume of an exercise event triggered by the user within the period of time, where the exercise volume may be calories, for example, 100 kcal or 1000 kJ, consumed by the user to execute the exercise event.

In other words, the model may be trained by using known blood glucose values within a historical time period and an exercise volume of a known exercise event that occurs within the historical time period, to obtain the exercise volume identification model. In this way, when the exercise volume identification model and the consecutive blood glucose values recorded within the period of time are known, the exercise volume of the exercise event that occurs within the time period may be obtained through identification.

The exercise type identification model may be a classification model, for example, a support vector machine (Support Vector Machine, SVM) model, a random forest model, or a logistic regression model. The model $X1(\cdot)$ may be represented by using the following Formula 18:

$$S1 = X1(g1, g2, ..., gn) \quad \text{Formula 18}$$

Herein, g1, g2, ..., gn represents a blood glucose feature extracted based on a plurality of blood glucose values within a period of time, for example, an average value or a root mean square, and S1 represents an exercise type of an exercise event triggered by the user within the period of time. For example, the exercise type may include high-intensity exercise, low-intensity exercise, and the like.

In other words, the model may be trained by using the known blood glucose values within the historical time period and an exercise type of the known exercise event that occurs within the historical time period, to obtain the exercise type identification model. In this way, when the exercise type identification model and the consecutive blood glucose values recorded within the period of time are known, the exercise type of the exercise event that occurs within the time period may be obtained through identification.

(b) Medication identification model

The medication identification model may be used to identify medication start time, a medication volume, and a medicine type of a medication event.

For example, the medication identification model may include a medication start time identification model, a medication volume identification model, and a medicine type identification model.

The medication start time identification model may identify the medication start time based on values of features such as variability, an average value, and a change rate of blood glucose values within a period of time. For example, when the variability and the average value of the blood glucose values of the user within the period of time satisfy a preset condition, it is determined that the medication start time is specified time that has a preset association with the period of time.

The preset condition may be determined based on a blood glucose feature of historical blood glucose data and medication start time of a historical medication event. For example, the preset condition may be a condition that is satisfied by features of blood glucose in most (for example, 80%) of historical medication events within a period of time before and after medication. For example, it is learned from the historical medication event and the historical blood glucose data that there is a high probability that a change rate of blood glucose within half an hour after the user starts to take medicine is greater than a threshold. Therefore, if it is identified that the change rate of the blood glucose of the user within half an hour is greater than the threshold, it is determined that the user has a medication event, where start time of the half hour is medication start time of the medication event.

The medication volume identification model may be a regression model, for example, an SVC model, a random forest model, or a linear regression model. The model $\Phi2(\cdot)$ may be represented by using the following Formula 19:

$$L2 = \Phi2(G) \quad \text{Formula } 19$$

G represents consecutive blood glucose values recorded within a period of time, and L2 represents a medication volume of a medication event triggered by the user within the period of time, where the medication volume may be a dose of medicine, for example, 1 u (1 unit) or 50 mg, taken by the user to execute the medication event.

In other words, the model may be trained by using known blood glucose values within a historical time period and a medication volume of a known medication event that occurs within the historical time period, to obtain the medication volume identification model. In this way, when the medication volume identification model and the consecutive blood glucose values recorded within the period of time are known, the medication volume of the medication event that occurs within the time period may be obtained through identification.

The medicine type identification model may be a classification model, for example, an SVM model, a random forest model, or a logistic regression model. The model $X2(\cdot)$ may be represented by using the following Formula 20:

$$S2 = X2(g1, g2, …, gn) \quad \text{Formula } 20$$

Herein, g1, g2, ..., gn represents a blood glucose feature extracted based on a plurality of blood glucose values within a period of time, for example, an average value or a root mean square, and S2 represents a medicine type of a medicine taken by the user in a medication event triggered by the user within the period of time, for example, metformin, acarbose, and rapid-acting insulin.

In other words, the model may be trained by using known blood glucose values within a historical time period and a medicine type of a known medication event that occurs within the historical time period, to obtain the medicine type identification model. In this way, when the medicine type identification model and the consecutive blood glucose values recorded within the period of time are known, the medicine type of the medication event that occurs within the time period may be obtained through identification.

(c) Diet identification model

The diet identification model may be used to identify medication start time, a meal volume, and a diet type of a diet event.

For example, the diet identification model may include a meal start time identification model, a meal volume identification model, and a diet type identification model.

The meal start time identification model may identify the meal start time based on values of features such as variability, an average value, and a change rate of blood glucose values within a period of time. For example, when the variability and the average value of the blood glucose values of the user within the period of time satisfy a preset condition, it is determined that the meal start time is specified time that has a preset association with the period of time.

The preset condition may be determined based on a blood glucose feature of historical blood glucose data and meal start time of a historical diet event. For example, the preset condition may be a condition that is satisfied by features of blood glucose in most (for example, 80%) of historical diet events within a period of time before and after a meal. For example, it is learned from the historical diet event and the historical blood glucose data that there is a high probability that an average blood glucose value of the user within one hour after half an hour of starting a meal exceeds 7.2 mmol/L. Therefore, if it is identified that the average blood glucose value of the user within one hour exceeds 7.2 mmol/L, it is determined that the user has a diet event, where a time point at which half an hour is backtracked from start time of the one hour is meal start time of the diet event.

The meal volume identification model may be a regression model, for example, an SVC model, a random forest model, or a linear regression model. The model $\Phi3(\cdot)$ may be represented by using the following Formula 21:

$$L3 = \Phi3(G) \quad \text{Formula 21}$$

G represents consecutive blood glucose values recorded within a period of time, and L3 represents a meal volume of a diet event triggered by the user within the period of time, where the meal volume may be calories, for example, 100 kcal or 1000 kJ, ingested by the user when the user executes the diet event.

In other words, the model may be trained by using known blood glucose values within a historical time period and a meal volume of a known diet event that occurs within the historical time period, to obtain the meal volume identification model. In this way, when the meal volume identification model and the consecutive blood glucose values recorded within the period of time are known, the meal volume of the diet event that occurs within the time period may be obtained through identification.

The diet type identification model may be a classification model, for example, an SVM model, a random forest model, or a logistic regression model. The model $X3(\cdot)$ may be represented by using the following Formula 22:

$$S3 = X3(g1, g2, ..., gn) \quad \text{Formula 22}$$

Herein, g1, g2, ... , gn represents a blood glucose feature extracted based on a plurality of blood glucose values within a period of time, for example, an average value or a root mean square, and S3 represents a diet type of a diet ingested by the user in a diet event triggered by the user within the period of time, for example, foods with a high glycemic index, foods with a medium glycemic index, and foods with a low glycemic index.

In other words, the model may be trained by using known blood glucose values within a historical time period and a diet type of a known diet event that occurs within the historical time period, to obtain the diet type identification model. In this way, when the diet type identification model and the consecutive blood glucose values recorded within the period of time are known, the diet type of the diet event that occurs within the time period may be obtained through identification.

(2) The exogenous event that may occur after the current time point may be obtained through prediction based on the work and rest rule of the user.

In this case, a daily work and rest rule of the user may be obtained by summarizing a large amount of information of the user within a period of time, to predict an exogenous event in advance. The large amount of information may include but is not limited to: a scheduled activity set by the user, data (for example, a breath, blood glucose, a heart rate, blood pressure, exercise time, and an exercise volume) collected by a plurality of sensors, browsing and search records of the user on an application, and the like.

For example, it may be determined, based on a large amount of exercise data of the user within a period of time, that the user has an exercise habit of jogging at 9:00 a.m. every day for half an hour. Therefore, an exercise event of the user at 9:00 a.m. every day may be obtained through identification based on the exercise habit.

It may be understood that the electronic device 100 may alternatively learn of the exogenous event in another manner. For example, the user may actively enter the exogenous event, including entering related information of the exogenous event.

For example, when the user enters an exercise event, the user may enter exercise start time, an exercise type, an exercise volume, and the like of the exercise event; when the user enters a medication event, the user may enter medication start time, a medicine type, a medication volume, and the like of the medication event; or when the user enters a diet event, the user may enter meal start time, a diet type, a meal volume, and the like of the diet event.

For example, the user may enter having a meal at 12:00 every day. For another example, the user may enter that a hypoglycemic medicine is taken at 6:00 p.m. on the previous day. For another example, the user may enter that running exercise needs to be performed at 9:00 a.m. tomorrow.

In some implementations, the user may change the exogenous event. For example, for the exercise event, the user may change the exercise start time, the exercise volume, the exercise type, and the like of the exercise event. For the medication event, the user may change the medication start time, the medication volume, the medicine type, and the like of the medication event. For the diet event, the user may change the meal start time, the meal volume, the diet type, and the like of the diet event.

In this way, the user may manually adjust the exogenous event used to predict future blood glucose, to enhance participation of the user. In addition, for the exogenous event manually corrected by the user, the predicted future blood glucose can be more accurate, and accuracy of predicting the future blood glucose can be improved.

**S702:** Obtain blood glucose values of the user within a period of time before the current time point.

For example, the blood glucose values of the user within the period of time (for example, fifth duration) before the current time point may be collected by using an electronic device (for example, the electronic device 200). The blood glucose values within the period of time may include a plurality of consecutive blood glucose values.

**S703:** Predict blood glucose values within a period of time after the current time point based on the exogenous event and the blood glucose values.

For example, the blood glucose values within the period of time (for example, sixth duration) after the current time point may be predicted based on the exogenous event and the blood glucose values by using the blood glucose prediction model.

Inputs of the blood glucose prediction model are the exogenous event and a historical blood glucose value, and an input is a future blood glucose value. The blood glucose prediction model is obtained through training within the period of time based on known blood glucose values within a historical period of time and a known exogenous event within a previous period of time. During training, the known blood glucose value and exogenous event within the previous period of time (for example, a first period of time) are input data of the model within the period of time, and known blood glucose values within a subsequent period of time (for example, a second period of time) are output data of the model within the period of time.

A manner of obtaining the exogenous event during model training may include: (1) obtaining through identification by using an event identification model; and (2) entering by the user. For a specific manner of obtaining the exogenous event, refer to related content in step S701. Details are not described herein again.

It can be learned that the blood glucose prediction model may predict a plurality of blood glucose values within a future period of time based on a plurality of blood glucose values within a period of time and an exogenous event within the period of time.

The blood glucose prediction model may be a time series forecasting model, for example, a conventional time series forecasting model like an autoregressive model or a linear regression model, or a machine learning model like a random forest model or a support vector regression (Support Vector Regression, SVR) model.

For example, the blood glucose prediction model $\Psi(\cdot)$ may be represented by using the following Formula 23:

$$G_{after} = \Psi(G_{before}, C) \quad \text{Formula 23}$$

Herein, $G_{before}$ represents blood glucose values of the user within a period of time before a time point T0, $G_{after}$ represents blood glucose values of the user within a period of time after the time point T0, and C represents an exogenous event that occurs within the period of time before the time point T0, where the exogenous event includes one or more of the following: an exercise event C1, a medication event C2, and a diet event C3. C1 may be represented by three parameters: exercise start time T1, an exercise volume L1, and an exercise type S1; C2 may be represented by three parameters: medication start time T2, a medication volume L2, and a medicine type S2; and C3 may be represented by meal start time T3, a meal volume L3, and a diet type S3.

In a process of training the blood glucose prediction model, both an input blood glucose value and an output blood glucose value of the blood glucose prediction model are collected blood glucose values. A process of training the model is specifically as follows: After blood glucose values and an exogenous event that are collected within a period of time are input into the blood glucose prediction model, a parameter of the blood glucose prediction model is adjusted, so that an output of the blood glucose prediction model is blood glucose values collected within preset duration after the period of time, and the blood glucose prediction model obtained after the parameter is adjusted is the blood glucose prediction model obtained through training.

In addition, during model training, when no exogenous event occurs within a period of time in which the blood glucose values input into the model exist, the input does not include the exogenous event. It can be learned that, the blood glucose prediction model $\Psi(\cdot)$ may alternatively be represented as: $G_{after} = \Psi(G_{before})$. An input of the blood glucose prediction model is blood glucose values $G_{before}$ of the user within the period of time before the time point T0, and an output is blood glucose values $G_{after}$ of the user within the period of time after the time point T0.

It can be learned that, for the blood glucose prediction model, blood glucose values within a future period of time can also be predicted by inputting only blood glucose values of the user within a period of time. However, if an exogenous event that occurs within the period of time is not input into the blood glucose prediction model, the predicted blood glucose values are not as accurate as blood glucose values obtained through prediction after the exogenous event is input. It can be learned that, the exogenous event of the user is also used as a factor for predicting blood glucose, so that accuracy of predicting the blood glucose can be improved.

In a process of predicting the future blood glucose by using the blood glucose prediction model, specifically, the obtained exogenous event and the collected blood glucose values within the period of time before the current time point are used as input parameters of the blood glucose prediction model and input into the blood glucose prediction model for prediction, and an output parameter of the blood glucose prediction model is the predicted blood glucose values within the period of time after the current time point.

When the exogenous event includes an exercise event (for example, a first exercise event), when the blood glucose prediction model is used to predict blood glucose, the input of the blood glucose prediction model includes exercise start time, an exercise volume, and an exercise type of the exercise event. When the exogenous event includes a medication event (for example, a first medication event), when the blood glucose prediction model is used to predict blood glucose, the input of the blood glucose prediction model includes medication start time, a medication volume, and a medicine type of the medication event. When the exogenous event includes a diet event (for example, a first diet event), when the blood glucose prediction model is used to predict blood glucose, the input of the blood glucose prediction model includes meal start time, a meal volume, and a diet type of the diet event.

In addition, it should be noted that, when the exogenous event is an event that does not occur after the current time point, when the blood glucose prediction model is used to predict blood glucose, blood glucose values obtained through prediction from the current time point to event start time of the exogenous event are obtained through prediction by using the blood glucose prediction model based on blood glucose data before the current time point, and blood glucose values after the event start time are obtained through prediction by using the blood glucose prediction model based on the blood glucose obtained through prediction before the event start time and the collected blood glucose values.

It may be understood that, when there is no exogenous event, blood glucose data within the period of time after the current time point may alternatively be obtained through prediction by using the blood glucose prediction model only based on the blood glucose data.

In some implementations, because the exogenous event causes a great blood glucose change of the user in short time, when the exogenous event is an event that has occurred before the current time point, before the exogenous event is obtained, whether the exogenous event needs to be identified and obtained may be determined in advance based on blood glucose data within the period of time before the current time point. For example, when a change rate of blood glucose within an adjacent time period (for example, 10 minutes) before the current time point is greater than a threshold (for example, a fifth threshold), the exogenous event that has occurred before the current time point may be triggered to be obtained. Further, the future blood glucose is predicted based on the exogenous event and the known blood glucose data. Otherwise, only the known blood glucose data may be used to predict the future blood glucose. In this way, the exogenous event that has occurred on the user may be obtained only when it is determined that blood glucose data of the user meets the preset condition, and the future blood glucose may be predicted by using the exogenous event and the blood glucose data.

In some implementations, after blood glucose within the period of time after the current time point is obtained through prediction by using the exogenous event and the blood glucose values within the period of time before the current time point, a blood glucose curve (for example, a fourth blood glucose curve) may be displayed. The blood glucose curve indicates a blood glucose change of the user within the period of time before the current time point, and a blood glucose change obtained through prediction within the period of time after the current time point by using the exogenous event and the blood glucose values within the period of time before the current time point. In this way, the user can learn of a past and future blood glucose change trend, manage and intervene a blood glucose change of the user, and avoid in time harm caused by hyperglycemia or hypoglycemia. For example, the blood glucose curve may be displayed by using the electronic device 100.

In some implementations, the exogenous event may be further marked on the displayed blood glucose curve. In this way, the blood glucose of the user may be associated with the exogenous event, so that the user controls, in time starting from the exogenous event, a blood glucose change of the user. This helps the user perform the exogenous event in a more scientific and healthier manner and improve a self-management capability of the user on blood glucose.

In some implementations, after the future blood glucose is obtained through prediction, the exogenous event may be further adjusted based on a user operation, the future blood glucose is repredicted based on an adjusted exogenous event and the blood glucose values, and an adjusted blood glucose curve (for example, a fifth blood glucose curve) is redisplayed. The adjusted blood glucose curve indicates a blood glucose change within the period of time before the current time point and a blood glucose change obtained through reprediction within the period of time after the current time point.

In other words, the user may manually adjust the exogenous event, and view the blood glucose curve obtained through prediction after the exogenous event is adjusted, to enhance participation of the user. In addition, an association between the exogenous event and the blood glucose may be further dynamically learned. When the user needs to plan a healthy exogenous event, the exogenous event may be adjusted on a premise that the future blood glucose fluctuates within a normal range, and the exogenous event obtained through adjustment is used as the healthy exogenous event planned by the user, to help the user more easily manage and control the blood glucose change of the user.

In some implementations, before the exogenous event is obtained, a blood glucose curve (for example, a sixth blood glucose curve) obtained through prediction when the exogenous event is not considered may be further displayed. The blood glucose curve indicates a blood glucose change of the user within the period of time before the current time point, and a blood glucose change obtained through prediction within the period of time after the current time point by using the blood glucose values within the period of time before the current time point. In this way, both the blood glucose curve obtained through prediction without considering the exogenous event and the blood glucose curve obtained through prediction when the exogenous event is considered may be displayed, to highlight a difference between blood glucose predictions obtained by considering the exogenous event and without considering the exogenous event in this method.

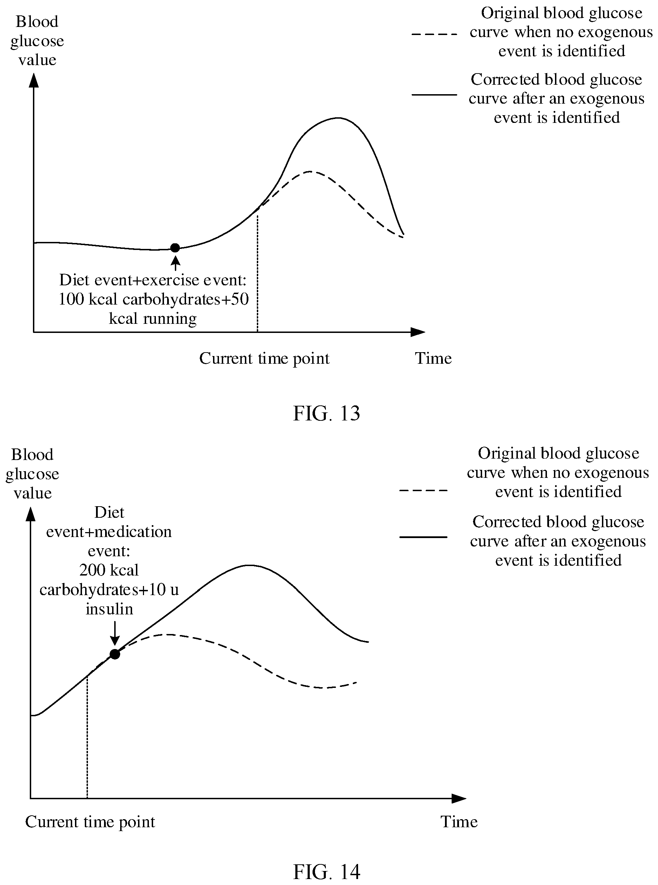

FIG. 13 and FIG. 14 are respectively diagrams of blood glucose curves of exogenous events in different cases.

FIG. 13 is an example diagram of blood glucose curves in a case in which the exogenous event is an event that has occurred currently.

As shown in FIG. 13, the exogenous event includes a diet event and an exercise event. The diet event is intake of 100 kcal carbohydrates, and the exercise event is running exercise that consumes 50 kcal calories. Occurrence time of the exogenous event is earlier than the current time point. A blood glucose curve before the current time point is drawn based on historical blood glucose values of the user. An original blood glucose curve (dashed line) after the current time point is directly drawn, when no exogenous event is identified, based on blood glucose values that are obtained through prediction by using the blood glucose prediction model and based on blood glucose values before the current time point. A corrected blood glucose curve (solid line) after the current time point is drawn, after an exogenous event is identified, based on blood glucose values that are obtained through prediction by using the blood glucose prediction model and based on the blood glucose values before the current time point and the exogenous event identified before the current time point. By comparing the original blood glucose curve and the corrected blood glucose curve after the current time point, it can be learned that blood glucose values on the blood glucose curve obtained through prediction when no exogenous event is identified are generally lower than blood glucose values on the blood glucose curve obtained through prediction after the exogenous event is identified. Accuracy of predicting future blood glucose without identifying the exogenous event is lower than accuracy of predicting future blood glucose when the exogenous event is identified and used.

FIG. 14 is an example diagram of blood glucose curves in a case in which the exogenous event is an event that has not occurred currently.

As shown in FIG. 14, the exogenous event includes a diet event and a medication event. The diet event is intake of 200 kcal carbohydrates, and the medication event is intake of 10 u insulin. Occurrence time of the exogenous event is later than a current time point. A blood glucose curve before the current time point is drawn based on historical blood glucose values of the user. An original blood glucose curve (dashed line) after the current time point is drawn based on blood glucose values that are obtained through prediction based on blood glucose values before the current time point. A corrected blood glucose curve (solid line) after the current time point is drawn based on blood glucose values that are obtained through prediction by using the blood glucose prediction model and based on the blood glucose values before the current time point and the exogenous event obtained through prediction after the current time point. In addition, the original blood glucose curve and the corrected blood glucose curve coincide between the current time point and the occurrence time point of the exogenous event. By comparing the original blood glucose curve and the corrected blood glucose curve after the current time point, it can be learned that blood glucose values on the blood glucose curve obtained through prediction when no exogenous event is identified are generally lower than blood glucose values on the blood glucose curve obtained through prediction after the exogenous event is identified. Accuracy of predicting future blood glucose values without identifying the exogenous event is lower than accuracy of predicting future blood glucose values when an exogenous event that may occur in the future is identified, and impact of the exogenous event on the blood glucose of the user is considered.

It can be learned from steps S701 to S703 that, when the future blood glucose is predicted, the exogenous event that can affect the blood glucose change is used as an external factor for predicting the blood glucose, so that accuracy of predicting the blood glucose can be improved. Further, the exogenous event is identified by using the event identification model and based on the work and rest rule of the user. This can reduce troubles of a manual input by the user, and avoid troubles of searching, and an incorrect or missing input of the user, so that a blood glucose prediction technology is more intelligent and comprehensive.

It may be understood that the blood glucose management methods in the foregoing four embodiments provided in this application may be integrated with each other. For example, when obtaining the meal time of the user through calculation, and displaying the meal time and the blood glucose value related to the meal time on the blood glucose curve, the electronic device 100 may further find, based on the meal volume and the meal speed of the diet event of the user, the standard blood glucose curve of the healthy people that have the same meal volume and meal speed as the user, and display the standard blood glucose curve when displaying the blood glucose curve of the user. Alternatively, the electronic device 100 displays the meal time and the like of the user on the blood glucose curve when obtaining the future blood glucose value through prediction and displaying a blood glucose curve including past and future blood glucose. It can be learned that a plurality of pieces of information may be simultaneously displayed to the user through the interaction interface of the electronic device, to help the user learn of the blood glucose status of the user from a plurality of aspects, so that the user can better manage and control the blood glucose change of the user starting from the exogenous event.

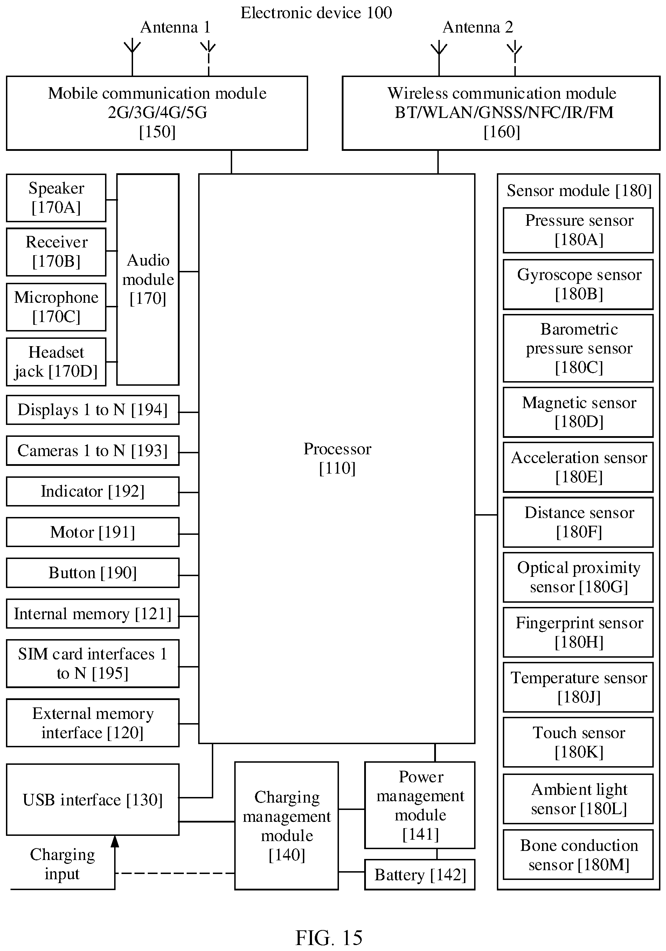

**FIG. 15 is a diagram of a hardware structure of the electronic device 100 according to an embodiment of this application.**

The electronic device 100 may be a mobile phone, a tablet computer, a desktop computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR) device, a virtual reality (virtual reality, VR) device, an artificial intelligence (artificial intelligence, AI) device, a wearable device, a vehicle-mounted device, a smart home device, and/or a smart city device. A specific type of the electronic device is not limited in embodiments of this application.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in embodiments of the present invention does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by using hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

**In some embodiments, the processor 110 may be configured to perform related operation steps in Embodiment 1 to Embodiment 4. For example, in Embodiment 1, the processor 110 may be configured to: obtain a fasting blood glucose value of a user, determine whether a diet event exists within a preset time period, determine meal time of the diet event, update the fasting blood glucose value, and the like. For another example, in Embodiment 2, the processor 110 may be configured to: determine a heart rate difference, determine whether the heart rate difference is greater than or equal to a threshold 1, calculate an exercise impact factor, and determine, based on the heart rate difference, the exercise impact factor, and an emotion and stress factor, whether the user starts a meal. In addition, the processor 110 may be further configured to: obtain meal-related data collected by a sensor during the meal of the user, calculate a meal volume and a meal speed, obtain an actual blood glucose curve and a standard blood glucose curve, and evaluate diet health of the user based on the actual blood glucose curve and the standard blood glucose curve. For another example, in Embodiment 3, the processor 110 may be configured to: after identifying that the user is to start exercise, obtain exercise reference data of the user before the exercise, calculate an exercise risk score and an exercise risk threshold, and determine exercise prompt information. The processor 110 may be further configured to: after the user starts the exercise, obtain exercise reference data of the user during the exercise, calculate an exercise risk score, and determine whether to input an exercise alarm. The processor 110 may be further configured to: after the user ends the exercise, obtain exercise reference data and exercise feedback information of single exercise of the user, determine an exercise evaluation report, and the like. For another example, in Embodiment 4, the processor 110 may be configured to: train a blood glucose prediction model, predict future blood glucose based on blood glucose data of the user and an exogenous event, and the like. For specific content, refer to related descriptions in Embodiment 1 to Embodiment 4. Details are not described herein again.**

The controller may generate an operation control signal based on an instruction operation code and a time sequence signal, to complete control over instruction fetching and instruction execution.

A memory may be further disposed in the processor 110, and is configured to store instructions and data. In some embodiments, the memory in the processor 110 is a cache. The memory may store instructions or data just used or cyclically used by the processor 110. If the processor 110 needs to use the instructions or the data again, the processor 110 may directly invoke the instructions or the data from the memory. This avoids repeated access, reduces waiting time of the processor 110, and improves system efficiency.

The charging management module 140 is configured to receive a charging input from the charger. The charger may be a wireless charger or a wired charger. In some embodiments of wired charging, the charging management module 140 may receive a charging input of a wired charger through the USB interface 130. In some embodiments of wireless charging, the charging management module 140 may receive a wireless charging input through a wireless charging coil of the electronic device 100. When charging the battery 142, the charging management module 140 may further supply power to the electronic device through the power management module 141.

The power management module 141 is configured to connect the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like. The power management module 141 may be configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same component.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution that is applied to the electronic device 100 and that includes 2G/3G/4G/5G or the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least a part of functional modules of the mobile communication module 150 may be disposed in the processor 110. In some embodiments, at least a part of functional modules of the mobile communication module 150 may be disposed in a same device as at least a part of modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 170A, the receiver 170B, or the like), or displays an image or a video through the display 194. In some embodiments, the modem processor may be an independent component. In some other embodiments, the modem processor may be independent of the processor 110, and is disposed in a same device as the mobile communication module 150 or another functional module.

The wireless communication module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processing module. The wireless communication module 160 receives an electromagnetic wave through the antenna 2, performs demodulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

**In some embodiments, the electronic device 100 may obtain, through the mobile communication module 150 or the wireless communication module 160, data, including blood glucose data, a heart rate, exercise data, and the like, collected by another device, for example, the electronic device 200 or the electronic device 300.**

In some embodiments, in the electronic device 100, the antenna 1 and the mobile communication module 150 are coupled, and the antenna 2 and the wireless communication module 160 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

The electronic device 100 implements a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs that execute program instructions to generate or change display information.

The display 194 is configured to display an image, a video, and the like. The display 194 includes a display panel. The display panel may be a liquid crystal display (liquid crystal display, LCD). The display panel may alternatively be an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like. In some embodiments, the electronic device may include one or N displays 194, where N is a positive integer greater than 1.

**In some embodiments, the display 194 may be configured to display a blood glucose curve, exercise prompt information of the user before exercise, an exercise alarm during the exercise, an exercise evaluation report after the exercise, and the like. For example, the blood glucose curve may be the curve shown in FIG. 3, FIG. 4, FIG. 5, FIG. 13, or FIG. 14.**

The electronic device 100 may implement an image shooting function through the ISP, the camera 193, the video codec, the GPU, the display 194, the application processor, and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise and brightness of the image. The ISP may further optimize parameters such as exposure and a color temperature of an image shooting scenario. In some embodiments, the ISP may be disposed in the camera 193.

The camera 193 is configured to capture a static image or a video. An optical image of an object is generated through the lens, and is projected onto the photosensitive element. The photosensitive element may be a charge-coupled device (charge-coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format like RGB or YUV. In some embodiments, the electronic device 100 may include one or N cameras 193, where N is a positive integer greater than 1.

**In some embodiments, the camera 193 may be configured to take a photo of food when the user is having a meal, so that the processor 110 determines, based on image shooting time, time at which the user starts the meal.**

The digital signal processor is configured to process a digital signal, and may process another digital signal in addition to the digital image signal. For example, when the electronic device 100 selects a frequency, the digital signal processor is configured to perform Fourier transformation on frequency energy.

The video codec is configured to compress or decompress a digital video. The electronic device 100 may support one or more video codecs. In this way, the electronic device 100 may play or record videos in a plurality of encoding formats, for example, moving picture experts group (moving picture experts group, MPEG)-1, MPEG-2, MPEG-3, and MPEG-4.

The NPU is a neural-network (neural-network, NN) computing processor, quickly processes input information by referring to a structure of a biological neural network, for example, by referring to a transmission mode between human brain neurons, and may further continuously perform self-learning. The NPU can implement applications such as intelligent cognition of the electronic device 100, for example, image recognition, speech recognition, and text understanding.

The internal memory 121 may include one or more random access memories (random access memories, RAMs) and one or more nonvolatile memories (nonvolatile memories, NVMs).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, for example, a 5th generation DDR SDRAM, generally referred to as DDR5 SDRAM), or the like. The nonvolatile memory may include a magnetic disk storage device and a flash memory (flash memory).

The flash memory may be classified, based on an operation principle, into an NOR flash, an NAND flash, a 3D NAND flash, and the like; may be classified, based on a quantity of electric potential levels of a cell, into a single-level cell (single-level cell, SLC), a multi-level cell (multi-level cell, MLC), a triple-level cell (triple-level cell, TLC), a quad-level cell (quad-level cell, QLC), and the like; or may be classified, based on storage specifications, into a universal flash storage (English: universal flash storage, UFS), an embedded multimedia card (embedded multimedia Card, eMMC), and the like.

The random access memory may be directly read and written by using the processor 110, may be configured to store executable programs (for example, machine instructions) in an operating system or another running program, and may be configured to store data of a user, data of an application, and the like.

The nonvolatile memory may also store the executable program, the data of the user, the data of the application, and the like, which may be loaded into the random access memory in advance for directly reading and writing by the processor 110.

In some embodiments, the internal memory 121 may be configured to store a blood glucose value, exercise data, a blood glucose curve, an event identification model, a blood glucose prediction model, and the like of the user.

The external memory interface 120 may be configured to connect to an external nonvolatile memory, to expand a storage capability of the electronic device 100. The external nonvolatile memory communicates with the processor 110 through the external memory interface 120, to implement a data storage function. For example, files such as music and a video are stored in the external nonvolatile memory.

The electronic device 100 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert an analog audio input into a digital audio signal. The audio module 170 may be further configured to code and decode an audio signal. In some embodiments, the audio module 170 may be disposed in the processor 110, or a part of functional modules of the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an electrical audio signal into a sound signal. When a call is answered or a voice message is received through the electronic device 100, the receiver 170B may be put close to a human ear to listen to a voice.

The microphone 170C, also referred to as a "mike" or a "mic", is configured to convert a sound signal into an electrical signal. When making a call or sending a voice message, the user may make a sound near the microphone 170C through the mouth of the user, to input a sound signal to the microphone 170C. At least one microphone 170C may be disposed in the electronic device 100.

The headset jack 170D is configured to connect to a wired headset.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may also calculate a touch location based on a detection signal of the pressure sensor 180A.

The gyroscope sensor 180B may be configured to determine a motion posture of the electronic device 100. In some embodiments, an angular velocity of the electronic device 100 around three axes (namely, axes x, y, and z) may be determined through the gyroscope sensor 180B. The gyroscope sensor 180B may be configured to implement image stabilization during image shooting. For example, when the shutter is pressed, the gyroscope sensor 180B detects an angle at which the electronic device 100 jitters, calculates, based on the angle, a distance for which a lens module needs to compensate, and allows the lens to cancel the jitter of the electronic device 100 through reverse motion, to implement image stabilization. The gyroscope sensor 180B may also be used in a navigation scenario and a somatic game scenario.

The barometric pressure sensor 180C is configured to measure barometric pressure. In some embodiments, the electronic device 100 calculates an altitude through a value of the barometric pressure measured by the barometric pressure sensor 180C, to assist in positioning and navigation.

The magnetic sensor 180D includes a Hall sensor. The electronic device 100 may detect opening and closing of a flip cover by using the magnetic sensor 180D. In some embodiments, when the electronic device 100 is a flip phone, the electronic device 100 may detect opening and closing of a flip cover based on the magnetic sensor 180D. Further, a feature like automatic unlocking of the flip cover is set based on a detected opening or closing state of the leather case or a detected opening or closing state of the flip cover.

The acceleration sensor 180E may detect accelerations in various directions (usually on three axes) of the electronic device 100. When the electronic device 100 is still, a magnitude and a direction of gravity may be detected. The acceleration sensor 180E may be further configured to identify a posture of the electronic device, and is used in an application like switching between a landscape mode and a portrait mode or a pedometer.

The distance sensor 180F is configured to measure a distance. The electronic device 100 may measure the distance in an infrared manner or a laser manner. In some embodiments, in an image shooting scenario, the electronic device 100 may measure a distance through the distance sensor 180F to implement quick focusing.

The optical proximity sensor 180G may include, for example, a light-emitting diode (LED) and an optical detector, for example, a photodiode. The light-emitting diode may be an infrared light-emitting diode. The electronic device 100 emits infrared light by using the light-emitting diode. The electronic device 100 detects infrared reflected light from a nearby object through the photodiode. When sufficient reflected light is detected, it may be determined that there is an object near the electronic device 100. When insufficient reflected light is detected, the electronic device 100 may determine that there is no object near the electronic device 100.

The ambient light sensor 180L is configured to sense ambient light brightness. The electronic device 100 may adaptively adjust brightness of the display 194 based on the sensed ambient light brightness. The ambient light sensor 180L may also be configured to automatically adjust white balance during photographing. The ambient light sensor 180L may also cooperate with the optical proximity sensor 180G to detect whether the electronic device 100 is in a pocket, to avoid an accidental touch.

The fingerprint sensor 180H is configured to collect a fingerprint. The electronic device 100 may use a feature of the collected fingerprint to implement fingerprint-based unlocking, application lock access, fingerprint-based photographing, fingerprint-based call answering, and the like.

The temperature sensor 180J is configured to detect a temperature. In some embodiments, the electronic device 100 executes a temperature processing strategy based on the temperature detected by the temperature sensor 180J. For example, when the temperature reported by the temperature sensor 180J exceeds a threshold, the electronic device 100 lowers performance of a processor located near the temperature sensor 180J, to reduce power consumption for thermal protection.

The touch sensor 180K is also referred to as a "touch component". The touch sensor 180K may be disposed on the display 194, and the touch sensor 180K and the display 194 form a touchscreen, which is also referred to as a "touchscreen". The touch sensor 180K is configured to detect a touch operation performed on or near the touch sensor. The touch sensor may transfer the detected touch operation to the application processor to determine a type of the touch event. A visual output related to the touch operation may be provided through the display 194. In some other embodiments, the touch sensor 180K may alternatively be disposed on a surface of the electronic device 100 at a location different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may alternatively be in contact with a body pulse to receive a blood pressure beating signal. In some embodiments, the bone conduction sensor 180M may alternatively be disposed in the headset, to obtain a bone conduction headset. The audio module 170 may obtain a speech signal through parsing based on the vibration signal that is of the vibration bone of the vocal-cord part and that is obtained by the bone conduction sensor 180M, to implement a speech function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt and a touch vibration feedback. For example, touch operations performed on different applications (for example, photographing and audio playing) may correspond to different vibration feedback effects. The motor 191 may also correspond to different vibration feedback effects for touch operations performed on different areas of the display 194.

Different application scenarios (for example, a time reminder, information receiving, an alarm clock, and a game) may also correspond to different vibration feedback effects. A touch vibration feedback effect may be further customized. **In some embodiments, the motor 191 may be configured to generate a vibration prompt when an exercise alarm is generated during exercise of the user.**

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100.

The electronic device may be a portable terminal device carrying iOS, Android, Microsoft, or another operating system, for example, a mobile phone, a tablet computer, or a wearable device; or may be a non-portable terminal device like a laptop (Laptop) having a touch-sensitive surface or a touch panel, or a desktop computer having a touch-sensitive surface or a touch panel. A software system of the electronic device 100 may use a layered architecture, an event-driven architecture, a microkernel architecture, a micro service architecture, or a cloud architecture. In embodiments of the present invention, an Android system of a layered architecture is used as an example to illustrate the software structure of the electronic device 100.

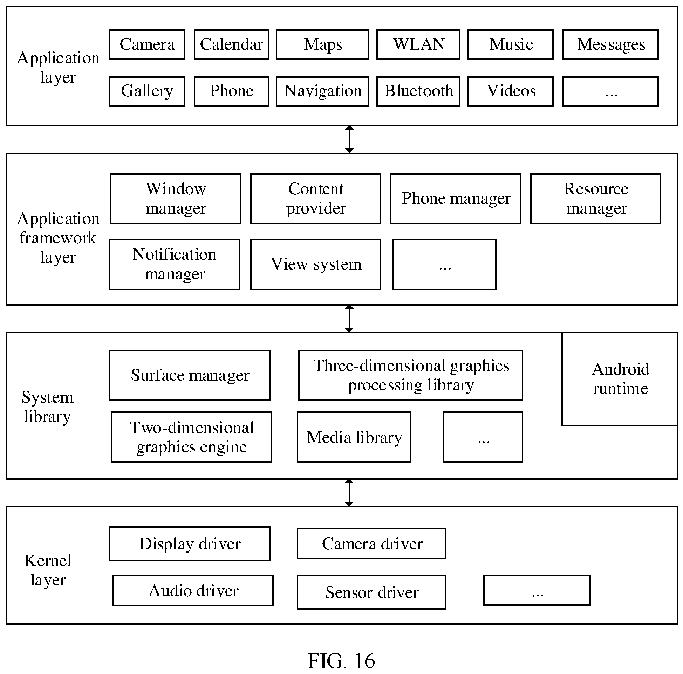

**FIG. 16 is a block diagram of a software structure of the electronic device 100 according to an embodiment of this application.**

In a layered architecture, software is divided into several layers, and each layer has a clear role and task. The layers communicate with each other through a software interface. In some embodiments, the Android system is divided into four layers: an application layer, an application framework layer, an Android runtime (Android runtime) and system library, and a kernel layer from top to bottom.

The application layer may include a series of application packages.

As shown in FIG. 16, the application packages may include applications such as Camera, Gallery, Calendar, Phone, Maps, Navigation, WLAN, Bluetooth, Music, Videos, and Messages.

The application framework layer provides an application programming interface (application programming interface, API) and a programming framework for an application at the application layer. The application framework layer includes some predefined functions.

As shown in FIG. 16, the application framework layer may include a window manager, a content provider, a view system, a phone manager, a resource manager, a notification manager, and the like.

The window manager is configured to manage a window program. The window manager may obtain a size of the display, determine whether there is a status bar, perform screen locking, take a screenshot, and the like.

The content provider is configured to: store and obtain data, and enable the data to be accessed by an application. The data may include a video, an image, an audio, calls that are made and answered, a browsing history and bookmarks, an address book, and the like.

The view system includes a visual control, for example, a control for displaying a text and a control for displaying an image. The view system may be configured to construct an application. A display interface may include one or more views. For example, a display interface including an SMS message notification icon may include a text display view and an image display view.

The phone manager is configured to provide a communication function of the electronic device 100, for example, management of a call status (including answering, declining, or the like).

The resource manager provides various resources such as a localized character string, an icon, an image, a layout file, and a video file for an application.

The notification manager enables an application to display notification information in the status bar, may be configured to convey a notification message, and may automatically disappear after a short pause without requiring a user interaction. For example, the notification manager is configured to notify download completion, give a message notification, and the like. The notification manager may alternatively be a notification that appears in a top status bar of the system in a form of graph or scroll bar text, for example, a notification of an application running on a background, or may be a notification that appears on the screen in a form of dialog window. For example, text information is displayed in the status bar, an alert tone is made, the electronic device vibrates, or the indicator blinks.

The Android runtime includes a core library and a virtual machine. The Android runtime is responsible for scheduling and management of the Android system.

The core library includes two parts: a function that needs to be called in Java language and a core library of Android.

The application layer and the application framework layer run on the virtual machine. The virtual machine executes java files of the application layer and the application framework layer as binary files. The virtual machine is configured to implement functions such as object lifecycle management, stack management, thread management, security and exception management, and garbage collection.

The system library may include a plurality of functional modules, for example, a surface manager (surface manager), a media library (Media Library), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playback and recording in a plurality of commonly used audio and video formats, and static image files. The media library may support a plurality of audio and video coding formats, for example, MPEG-4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

The kernel layer is a layer between hardware and software. The kernel layer includes at least a display driver, a camera driver, an audio driver, and a sensor driver.

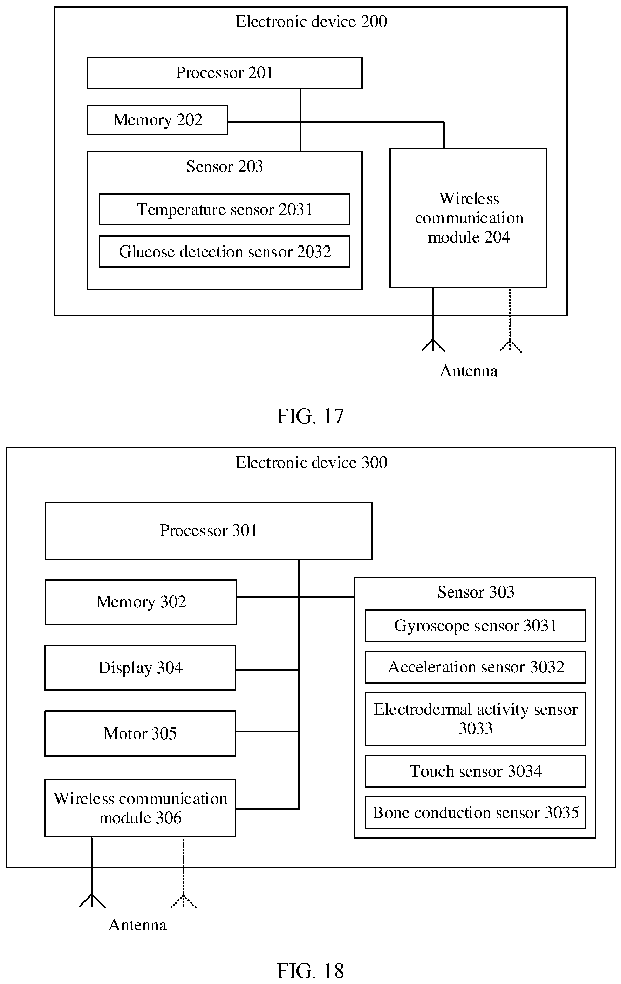

**FIG. 17 is a diagram of a hardware structure of the electronic device 200 according to an embodiment of this application.**

**The electronic device 200 is configured to measure blood glucose of a user. During specific implementation, the electronic device 200 may measure a tissue fluid glucose concentration (tissue fluid glucose), and then obtain, through calculation, a plasma (blood) glucose concentration (blood glucose).**

As shown in FIG. 17, the electronic device 200 includes a processor 201, a memory 202, a sensor 203, a wireless communication module 204, and the like.

It may be understood that the structure shown in embodiments of the present invention does not constitute a specific limitation on the electronic device. In some other embodiments of this application, the electronic device may include more or fewer components than those shown in the figure, or some components may be combined, or some components may be split, or different component arrangements may be used. The components shown in the figure may be implemented by using hardware, software, or a combination of software and hardware.

The processor 201 may include one or more processing units. For example, the processor 201 may be a modem processor, a digital signal processor, a controller, a baseband processor, a neural-network processing unit, and/or the like. Different processing units may be independent components, or may be integrated into one or more processors. The processor 201 may alternatively be referred to as a microcontroller processing unit.

The controller may generate an operation control signal based on an instruction operation code and a time sequence signal, to complete control over instruction fetching and instruction execution.

A memory may be further disposed in the processor 201, and is configured to store instructions and data. In some embodiments, the memory in the processor 201 is a cache. The memory may store instructions or data just used or cyclically used by the processor 201. If the processor 201 needs to use the instructions or the data again, the processor 201 may directly invoke the instructions or the data from the memory. This avoids repeated access, reduces waiting time of the processor 201, and improves system efficiency.

The wireless communication module 204 may provide a wireless communication solution that is applied to the electronic device and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 204 may be one or more components integrating at least one communication processing module. The wireless communication module 204 receives an electromagnetic wave through an antenna, performs frequency modulation and filtering processing on an electromagnetic wave signal, and sends a processed signal to the processor 201. The wireless communication module 204 may further receive a to-be-sent signal from the processor 201, perform frequency modulation and amplification on the signal, and convert the signal into an electromagnetic wave for radiation through the antenna.

The memory 202 may include one or more random access memories and one or more nonvolatile memories.

The nonvolatile memory may include a magnetic disk storage device and a flash memory.

The random access memory may be directly read and written by the processor 201, may be configured to store an executable program (for example, machine instructions) of an operating system or another running program, and may be further configured to store data of a user, data of an application, and the like.

The nonvolatile memory may also store an executable program, data of a user, data of an application, and the like, which may be loaded into the random access memory in advance for directly reading and writing by the processor 201.

A temperature sensor 2031 is configured to detect a temperature. In some embodiments, the electronic device 100 determines a skin temperature of the user and/or an ambient temperature by using the temperature detected by the temperature sensor 2031.

A glucose detection sensor 2032 is configured to detect a glucose concentration. In some embodiments, the glucose detection sensor 2032 may determine the glucose concentration by detecting consumption of oxygen under a catalytic action of glucose oxidase or $H_2O_2$ generated by a glucose oxidation reaction in a tissue fluid. In some embodiments, the glucose detection sensor 2032 connects the glucose oxidase to an electrode surface by using an electronic medium like a nanomaterial, metal osmium, ferrocene, or benzene quinone, then implements electron transfer through a series of oxidation-reduction reactions, and further determines the glucose concentration.

**In a process in which the user measures blood glucose by using the electronic device 200, the user may implant the electronic device 200 into a subcutaneous tissue, and the electronic device 200 measures the tissue fluid glucose concentration by using an electrode of the glucose detection sensor 2032, to determine the blood glucose concentration of the user.**

**In some embodiments, the electronic device 200 may send a collected blood glucose value to another device, for example, the electronic device 100, through the wireless communication module 204. The memory 202 may be configured to store the blood glucose value collected by the electronic device 200.**

It should be understood that FIG. 17 merely shows an example of the hardware structure of the electronic device 200. In another embodiment of this application, the electronic device 200 may include more or fewer components. This is not limited in embodiments of this application.

**FIG. 18 is a diagram of a hardware structure of the electronic device 300 according to an embodiment of this application.**

As shown in FIG. 18, the electronic device 300 may include a processor 301, a memory 302, a sensor 303, a display 304, a motor 305, and a wireless communication module 306. For the processor 301, refer to the text description of the processor 110. Details are not described herein again. For the memory 302, refer to the text description of the internal memory 121. Details are not described herein again. The sensor 303 may include a gyroscope sensor 3031, an acceleration sensor 3032, an electrodermal activity sensor 3033, a touch sensor 3034, and a bone conduction sensor 3035. For the gyroscope sensor 3031, refer to the text description of the gyroscope sensor 180B. Details are not described herein again. For the acceleration sensor 3032, refer to the text description of the acceleration sensor 180E. Details are not described herein again. For the touch sensor 3034, refer to the text description of the touch sensor 180K. Details are not described herein again. For the bone conduction sensor 3035, refer to the text description of the bone conduction sensor 180M. Details are not described herein again. The electrodermal activity sensor 3033 is configured to measure electrodermal activity data of the user, and the data is used to reflect a change of stress and emotion of the user. For the display 304, refer to the text description of the display 194. Details are not described herein again. For the motor 305, refer to the text description of the motor 191. Details are not described herein again. For the wireless communication module 306, refer to the text description of the wireless communication module 160. Details are not described herein again.

**In some embodiments, the electronic device 300 may send, through the wireless communication module 306, data collected by the sensor 303 to another electronic device, for example, the electronic device 100, obtain, through the wireless communication module 306, a blood glucose curve sent by the another electronic device, for example, the electronic device 100, and display the blood glucose curve through the display 304. The memory 302 may be configured to store the data collected by the sensor 303.**

It should be understood that FIG. 18 merely shows an example of the hardware structure of the electronic device 300. In another embodiment of this application, the electronic device 300 may include more or fewer components. This is not limited in embodiments of this application.

It should be understood that the steps in the foregoing method embodiments may be completed by using an integrated logic circuit of hardware in the processor or instructions in a form of software. The steps of the methods disclosed with reference to embodiments of this application may be directly performed by a hardware processor, or may be performed by using a combination of hardware in the processor and a software module.

This application further provides an electronic device. The electronic device may include a memory and a processor. The memory may be configured to store a computer program. The processor may be configured to invoke the computer program in the memory, so that the electronic device performs the method performed by the electronic device 100, the electronic device 200, or the electronic device 300 in any one of the foregoing embodiments.

This application further provides a chip system. The chip system includes at least one processor, configured to implement functions in the method performed by the electronic device 100, the electronic device 200, or the electronic device 300 in any one of the foregoing embodiments.

In a possible design, the chip system further includes a memory, the memory is configured to store program instructions and data, and the memory is located inside or outside the processor.

The chip system may include a chip, or may include a chip and another discrete component.

Optionally, there may be one or more processors in the chip system. The processor may be implemented by using hardware, or may be implemented by using software. When the processor is implemented by using the hardware, the processor may be a logic circuit, an integrated circuit, or the like. When the processor is implemented by using the software, the processor may be a general-purpose processor, and is implemented by reading software code stored in the memory.

Optionally, there may also be one or more memories in the chip system. The memory may be integrated with the processor, or may be disposed separately from the processor. This is not limited in embodiments of this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in embodiments of this application.

For example, the chip system may be a field programmable gate array (field programmable gate array, FPGA), an application-specific integrated circuit (application-specific integrated circuit, ASIC), a system on chip (system on chip, SoC), a central processing unit (central processing unit, CPU), a network processor (network processor, NP), a digital signal processor (digital signal processor, DSP), a microcontroller unit (microcontroller unit, MCU), a programmable logic device (programmable logic device, PLD), or another integrated chip.

This application further provides a computer program product. The computer program product includes a computer program (which may also be referred to as code or instructions). When the computer program is run, a computer is enabled to perform the method performed by the electronic device 100, the electronic device 200, or the electronic device 300 in any one of the foregoing embodiments.

This application further provides a computer-readable storage medium. The computer-readable storage medium stores a computer program (which may also be referred to as code or instructions). When the computer program is run, a computer is enabled to perform the method performed by the electronic device 100, the electronic device 200, or the electronic device 300 in any one of the foregoing embodiments.

It should be understood that the processor in embodiments of this application may be an integrated circuit chip, and has a signal processing capability. During implementation, steps in the foregoing method embodiments may be implemented by using a hardware integrated logic circuit in the processor, or by using instructions in a form of software. The processor may be a general-purpose processor, a digital signal processor (digital signal processor, DSP), an application-specific integrated circuit (Application-specific integrated circuit, ASIC), a field programmable gate array (field programmable gate array, FPGA) or another programmable logic device, a discrete gate or a transistor logic device, or a discrete hardware component. The processor may implement or perform the methods, the steps, and logical block diagrams that are disclosed in embodiments of this application. The general-purpose processor may be a microprocessor, or the processor may be any conventional processor or the like. The steps of the methods disclosed with reference to embodiments of this application may be directly performed by a hardware decoding processor, or may be performed by using a combination of hardware in the decoding processor and a software module. The software module may be located in a mature storage medium in the art, like a random access memory, a flash memory, a read-only memory, a programmable read-only memory, an electrically erasable programmable memory, or a register. The storage medium is located in the memory, and a processor reads information in the memory and completes the steps in the foregoing methods in combination with hardware of the processor.

In addition, an embodiment of this application further provides an apparatus. The apparatus may be specifically a component or a module, and the apparatus may include one or more processors and memories that are connected to each other. The memory is configured to store a computer program. When the computer program is executed by one or more processors, the apparatus is enabled to perform the methods in the foregoing method embodiments.

The apparatus, the computer-readable storage medium, the computer program product, or the chip provided in embodiments of this application is configured to perform the corresponding method provided above. Therefore, for beneficial effects that can be achieved, refer to beneficial effects in the corresponding method provided above. Details are not described herein again.

The implementations of this application may be randomly combined to achieve different technical effects.

All or a part of the foregoing embodiments may be implemented by using software, hardware, firmware, or any combination thereof. When software is used to implement the embodiments, all or a part of the embodiments may be implemented in a form of a computer program product. The computer program product includes one or more computer instructions. When the computer program instructions are loaded and executed on a computer, the procedure or functions according to this application are all or partially generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (solid state disk, SSD)), or the like.

Persons of ordinary skill in the art may understand that all or a part of the processes of the methods in embodiments may be implemented by a computer program instructing related hardware. The program may be stored in a computer-readable storage medium. When the program is executed, the processes of the methods in embodiments may be performed. The foregoing storage medium includes any medium that can store program code, for example, a ROM, a random access memory RAM, a magnetic disk, or an optical disc.

In conclusion, the foregoing descriptions are merely embodiments of the technical solutions of the present invention, but are not intended to limit the protection scope of the present invention. Any modification, equivalent replacement, or improvement made according to the disclosure of the present invention shall fall within the protection scope of the present invention.

**Claims**

1. A blood glucose management method, wherein the method is applied to an electronic device, and the method comprises:

   obtaining a fasting blood glucose value;
   if blood glucose values of a user within a first time period exceed the fasting blood glucose value by a first threshold, determining first meal time based on the blood glucose values within the first time period; and
   displaying the first meal time and/or a blood glucose value related to the first meal time on a displayed first blood glucose curve, wherein the first blood glucose curve indicates a blood glucose change of the user within the first time period.

2. The method according to claim 1, wherein the blood glucose value related to the first meal time comprises one or more of the following: a blood glucose value at the first meal time, and a blood glucose value at a time point first duration before or after the first meal time.

3. The method according to claim 1 or 2, wherein the first meal time is between a first time point and a second time point, the first time point is a time point of a blood glucose peak within the first time period, the second time point is before the first time point, and duration between the second time point and the first time point is second duration; and
   the blood glucose value at the first meal time is greater than the fasting blood glucose value, and between the first time point and the second time point, the first meal time is a $1^{st}$ time point at which a blood glucose rising rate of the user is greater than a second threshold.

4. The method according to any one of claims 1 to 3, wherein

   the fasting blood glucose value is determined based on blood glucose values of the user within a second time period, and the fasting blood glucose value is a lower quartile of the blood glucose values within the second time period, or the fasting blood glucose value is a lower quartile of blood glucose values between two meals of the user within the second time period; or
   the fasting blood glucose value is determined based on second meal time, the second meal time is before the first meal time, and the fasting blood glucose value is an average blood glucose value within third duration before the second meal time.

5. The method according to any one of claims 1 to 4, comprising:
   displaying a second blood glucose curve and/or a third blood glucose curve, wherein the second blood glucose curve indicates the fasting blood glucose value on each of N days, and the third blood glucose curve indicates a blood glucose value related to the first meal time on each of the N days, wherein N≥1 and N is an integer.

6. The method according to any one of claims 1 to 5, wherein the method further comprises:

   modifying the first meal time based on a user operation; and
   displaying, on the first blood glucose curve, the modified first meal time and/or a blood glucose value related to the modified first meal time.

7. The method according to any one of claims 1 to 6, wherein the method further comprises:
   displaying calories of food and/or a proportion of each nutrient.

8. A blood glucose management method, wherein the method is applied to an electronic device, and the method comprises:

determining a meal volume and a meal speed of a user during a meal;

obtaining an actual blood glucose curve of the user, wherein the actual blood glucose curve indicates a blood glucose change of the user during the meal;

obtaining a standard blood glucose curve of healthy people, wherein the standard blood glucose curve indicates a blood glucose change of the healthy people during a meal with the meal volume and the meal speed; and

evaluating diet health of the user based on the actual blood glucose curve and the standard blood glucose curve, wherein a larger difference between the actual blood glucose curve and the standard blood glucose curve indicates an unhealthier diet of the user, and a smaller difference between the actual blood glucose curve and the standard blood glucose curve indicates a healthier diet of the user.

9. The method according to claim 8, wherein obtaining the standard blood glucose curve of the healthy people specifically comprises:

obtaining blood glucose curves of a plurality of members in the healthy people, wherein the blood glucose curve indicates a blood glucose change of the member during the meal with the meal volume and the meal speed; and

determining the standard blood glucose curve of the healthy people based on the blood glucose curves of the plurality of members.

10. The method according to claim 9, wherein determining the standard blood glucose curve of the healthy people based on the blood glucose curves of the plurality of members specifically comprises:

performing stretching or compression transformation on the blood glucose curves of the plurality of members in time domain, and/or performing stretching or compression transformation on the blood glucose curves of the plurality of members in amplitude, so that average values of the blood glucose curves of the plurality of members in time domain and amplitude are equal; and

performing point-by-point average value calculation on transformed blood glucose curves of the plurality of members, and determining a curve formed by connecting the average values as the standard blood glucose curve.

11. The method according to any one of claims 8 to 10, wherein the method further comprises:

obtaining meal-related data of the user during the meal, wherein the meal-related data comprises heart rate data, exercise data, and emotion and stress data; and

determining the meal volume and the meal speed of the user during the meal specifically comprises:

inputting the meal-related data of the user during the meal into a meal model, to obtain, through identification, the meal volume and the meal speed of the user during the meal, wherein the meal model is obtained through training based on meal-related data during a meal with a known meal volume and meal speed.

12. The method according to claim 11, wherein before obtaining the meal-related data of the user during the meal, the method further comprises:

detecting that the user confirms an operation of starting the meal, or detecting that data collected by a sensor satisfies a preset condition.

13. The method according to claim 12, wherein the data collected by the sensor indicates a heart rate, exercise, and emotion and stress of the user, and the preset condition comprises: a difference between the heart rate of the user and a resting heart rate is still greater than a third threshold after impact of the exercise, and the emotion and stress is excluded.

14. The method according to any one of claims 8 to 13, wherein evaluating the diet health of the user based on the actual blood glucose curve and the standard blood glucose curve specifically comprises:

separately extracting features of the actual blood glucose curve and the standard blood glucose curve, wherein the features comprise one or more of the following: a maximum value of a single blood glucose change, non-stationary blood glucose time, quantities of blood glucose values in different blood glucose change rate categories, a quantity of blood glucose fluctuation times, time and amplitude of the single blood glucose change, and a curve envelope; and

evaluating the diet health of the user based on a difference between one or more features of the actual blood glucose curve and the one or more features of the standard blood glucose curve, wherein a larger difference indicates an unhealthier diet of the user, and a smaller difference indicates a healthier diet of the user.

15. The method according to claim 14, wherein the method further comprises:
calculating an overall health score of the user based on the difference during each of N meals of the user within a third time period, wherein a larger overall health score indicates an unhealthier diet of the user within the third time period, and a smaller overall health score indicates a healthier diet of the user within the third time period.

16. The method according to any one of claims 8 to 15, wherein the method further comprises:
displaying the actual blood glucose curve and the standard blood glucose curve.

17. A blood glucose management method, wherein the method is applied to an electronic device, and the method comprises:

obtaining first exercise reference data of a user before exercise;
outputting exercise prompt information based on the first exercise reference data, wherein the exercise prompt information indicates a recommendation for the exercise of the user;
obtaining second exercise reference data of the user during the exercise;
outputting an exercise alarm if the second exercise reference data reflects that an exercise risk of the user exceeds a fourth threshold;
obtaining third exercise reference data of the user after the exercise ends; and
outputting an exercise evaluation report of the exercise of the user based on the third exercise reference data, wherein the exercise evaluation report is used to evaluate impact of the exercise on the user, wherein
the first exercise reference data, the second exercise reference data, and the third exercise reference data comprise blood glucose and a parameter used to reflect a vital sign of the user.

18. The method according to claim 17, wherein before obtaining the first exercise reference data of the user before the exercise, the method further comprises:
detecting an operation entered by the user for indicating that the exercise is to start, or detecting that current time reaches exercise time preset by the user.

19. The method according to claim 17 or 18, wherein outputting the exercise prompt information based on the first exercise reference data specifically comprises:

performing weighted summation on levels of all data in the first exercise reference data to obtain a first exercise risk score through calculation, wherein a larger deviation of a data value of data from a normal range indicates a higher level of the data, wherein
when the first exercise risk score$<\lambda\cdot$TH, the exercise prompt information is that exercise is recommended;
when TH$>$the first exercise risk score$>\lambda\cdot$TH, the exercise prompt information is an exercise precaution or an optimized exercise scheme; or
when the first exercise risk score$>$TH, the exercise prompt information is that exercise is not recommended, wherein TH is determined based on basic information of the user and a blood glucose feature that reflects a blood glucose status of the user within a period of time.

20. The method according to any one of claims 17 to 19, wherein after obtaining the second exercise reference data of the user during the exercise, the method further comprises:
determining a second exercise risk score based on the second exercise reference data, wherein the second risk score indicates an exercise risk during the exercise.

21. The method according to claim 20, wherein the second exercise risk score is obtained through calculation according to the following formula:

$$G=\gamma(t)\sum c_i\, z_i,$$

wherein
$z_i$ represents a level of an $i^{th}$ piece of exercise reference data in the second exercise reference data, $c_i$ represents a weight of the $i^{th}$ piece of exercise reference data, and $\gamma(t)$ represents a time attenuation factor, wherein the time attenuation factor is a function that monotonically increases with time t, a larger deviation of a data value of the $i^{th}$ piece of exercise reference data from a normal range indicates a higher level of the $i^{th}$ piece of exercise reference data, a higher second exercise risk score indicates a higher exercise risk, and a lower second exercise risk score indicates a lower exercise risk.

22. The method according to any one of claims 17 to 21, wherein after outputting the exercise alarm, the method further comprises:
outputting the exercise alarm again after fourth duration starting when the user ends the exercise.

23. The method according to any one of claims 17 to 22, wherein after the user ends the exercise, the method further comprises:

obtaining exercise feedback information of the user; and
outputting the exercise evaluation report of the exercise of the user based on the third exercise reference data specifically comprises:
outputting the exercise evaluation report of the exercise based on the exercise feedback information and the third exercise reference data, wherein the exercise evaluation report further comprises a recommendation for next exercise that is determined based on the exercise feedback information.

24. The method according to any one of claims 17 to 23, wherein outputting the exercise evaluation report of the exercise of the user based on the third exercise reference data specifically comprises:
performing weighted summation on levels of all data in the third exercise reference data to calculate an exercise impact score, wherein a larger deviation of a data value of data in the third exercise reference data from a normal range indicates a higher level of the data, a higher exercise impact score indicates greater impact of the exercise on the user, and a lower exercise impact score indicates smaller impact of the exercise on the user.

25. A blood glucose management method, wherein the method is applied to an electronic device, and the method comprises:

identifying a first exogenous event by using an event identification model, or predicting the first exogenous event based on a work and rest rule of a user, wherein the event identification model is obtained through training based on a historical exogenous event and a historical blood glucose value of the user, and the first exogenous event comprises one or more of the following: a diet event, a medication event, and an exercise event;
obtaining blood glucose values of the user within fifth duration before a current time point; and
predicting blood glucose values within sixth duration after the current time point based on the first exogenous event and the blood glucose values within the fifth duration.

26. The method according to claim 25, wherein predicting the blood glucose values within the sixth duration after the current time point based on the first exogenous event and the blood glucose values within the fifth duration specifically comprises:
predicting the blood glucose values within the sixth duration after the current time point by using a blood glucose prediction model and based on the first exogenous event and the blood glucose values within the fifth duration, wherein an input of the blood glucose prediction model is an exogenous event and the historical blood glucose value, an output is a future blood glucose value, the blood glucose prediction model is obtained through training based on known blood glucose values within a historical first time period, a known exogenous event within the first time period, and known blood glucose values within a historical second time period, and the first time period is a time period adjacent to and before the second time period.

27. The method according to claim 26, wherein the event identification model comprises an exercise identification model, a medication identification model, and a diet identification model;

the exercise identification model is used to identify exercise start time, an exercise volume, and an exercise type of the exercise event, the medication identification model is used to identify medication start time, a medication volume, and a medicine type of the medication event, and the diet identification model is used to identify meal start time, a meal volume, and a diet type of the diet event; and
if the first exogenous event comprises a first exercise event obtained through identification by using the exercise identification model, when the blood glucose prediction model is used to predict a blood glucose value, the input of the blood glucose prediction model comprises exercise start time, an exercise volume, and an exercise type of the first exercise event;

if the first exogenous event comprises a first medication event obtained through identification by using the medication identification model, when the blood glucose prediction model is used to predict a blood glucose value, the input of the blood glucose prediction model comprises medication start time, a medication volume, and a medicine type of the first medication event; or

if the first exogenous event comprises a first diet event obtained through identification by using the diet identification model, when the blood glucose prediction model is used to predict a blood glucose value, the input of the blood glucose prediction model comprises meal start time, a meal volume, and a diet type of the first diet event.

28. The method according to any one of claims 25 to 27, wherein the method further comprises:
displaying a fourth blood glucose curve, wherein the fourth blood glucose curve indicates a blood glucose change of the user within a period of time before the current time point, and a blood glucose change obtained through prediction based on the first exogenous event and the blood glucose values within the fifth duration within a period of time after the current time point.

29. The method according to claim 28, wherein the method further comprises:
marking the first exogenous event on the fourth blood glucose curve.

30. The method according to any one of claims 25 to 29, wherein the method further comprises:

adjusting the first exogenous event based on a user operation;
repredicting blood glucose values within the sixth duration based on the adjusted first exogenous event and the blood glucose values within the fifth duration; and
displaying a fifth blood glucose curve, wherein the fifth blood glucose curve indicates the blood glucose change of the user within the period of time before the current time point, and a blood glucose change obtained through reprediction within the period of time after the current time point.

31. The method according to any one of claims 25 to 30, wherein before identifying the first exogenous event by using the event identification model, the method further comprises:
displaying a sixth blood glucose curve, wherein the sixth blood glucose curve indicates the blood glucose change of the user within the period of time before the current time point, and a blood glucose change obtained through prediction within the period of time after the current time point based on the blood glucose values within the fifth duration.

32. The method according to any one of claims 25 to 31, wherein before identifying the first exogenous event by using the event identification model, or predicting the first exogenous event based on the work and rest rule of the user, the method further comprises:
determining that a blood glucose change rate of the user within seventh duration before the current time point is greater than a fifth threshold.

33. An electronic device, comprising a memory, one or more processors, and one or more programs, wherein when the one or more processors execute the one or more programs, the electronic device is enabled to implement the method according to any one of claims 1 to 7, 8 to 16, 17 to 24, and 25 to 32.

34. A computer-readable storage medium, comprising instructions, wherein when the instructions are run on an electronic device, the electronic device is enabled to perform the method according to any one of claims 1 to 7, 8 to 16, 17 to 24, and 25 to 32.

35. A computer program product, wherein when the computer program product runs on a computer, the computer is enabled to perform the method according to any one of claims 1 to 7, 8 to 16, 17 to 24, and 25 to 32.

**Communication system 1000**

Electronic device 300

Connect

Connect

Connect

Electronic device 100

Electronic device 200

FIG. 1

```
┌─────────────────────────────────────────────────┐
│     Obtain a fasting blood glucose value of a user   │───∼ S101
└─────────────────────────────────────────────────┘
                        │
                        ▼
                   ╱─────────╲
                  ╱  Determine,  ╲
                 ╱ based on the fasting blood ╲
                ╱ glucose value, whether there is a diet ╲───∼ S102
                ╲  event within a preset time  ╱
                 ╲        period       ╱
                   ╲─────────╱
                        │
                        ▼
┌─────────────────────────────────────────────────┐
│         Determine meal time of the diet event       │───∼ S103
└─────────────────────────────────────────────────┘
                        │
                        ▼
┌ ─ ─ ─ ─ ─ ─ ─ ─ ─ ─ ─ ─ ─ ─ ─ ─ ─ ─ ─ ─ ─ ─ ─ ┐
│      Modify the meal time based on a user operation   │───∼ S104
└ ─ ─ ─ ─ ─ ─ ─ ─ ─ ─ ─ ─ ─ ─ ─ ─ ─ ─ ─ ─ ─ ─ ─ ┘
                        │
                        ▼
┌─────────────────────────────────────────────────┐
│ Update the fasting blood glucose value of the user based on │───∼ S105
│                   the meal time                  │
└─────────────────────────────────────────────────┘
                        │
                        ▼
┌─────────────────────────────────────────────────┐
│  Display a blood glucose curve formed by connecting blood │
│  glucose values of the user at a plurality of time points, and │───∼ S106
│   display, on the curve, meal time and/or a blood glucose │
│          value related to the meal time        │
└─────────────────────────────────────────────────┘
```

FIG. 2

FIG. 3

Fasting blood glucose value
before breakfast (mmol/L)

(a)

Blood glucose value obtained
1 h after breakfast (mmol/L)

(b)

Blood glucose value obtained
2 h after breakfast (mmol/L)

(c)

Blood glucose value obtained
3 h after breakfast (mmol/L)

(d)

FIG. 4

EP 4 625 428 A1

Breakfast:
Total calories: 272 kcal
Protein: 9 grams
Fat: 12 grams
Carbohydrates: 36 grams
Dietary fibers: 2 grams

FIG. 5

| Obtain meal-related data collected by a sensor during a meal of a user | S201 |

| Calculate a meal volume and a meal speed of the user based on the meal-related data collected by the sensor | S202 |

| Obtain an actual blood glucose curve of the user under the meal volume and the meal speed | S203 |

| Obtain blood glucose curves of a plurality of members having a same meal volume and meal speed in healthy people | S204 |

| Determine a standard blood glucose curve of the healthy people based on the plurality of blood glucose curves | S205 |

| Evaluate diet health of the user based on the actual blood glucose curve and the standard blood glucose curve | S206 |

FIG. 6

Obtain a current heart rate value of a
user, and determine a heart rate
difference between the current heart rate
value and a resting heart rate value of the
user

S301

Determine
whether the heart rate
difference is greater than or
equal to a
threshold 1

S302

Obtain exercise data of the user before a
current time point

S303

Calculate an exercise impact factor of a
meal of the user based on the exercise
data

S304

Obtain an emotion and stress factor of
the meal of the user

S305

Determine
whether the heart rate
difference is still greater than or
equal to a threshold 2 after impact of
the exercise impact factor and
the emotion and stress
factor is eliminated

S306

Determine that the user
starts the meal

S307

Determine that the user
does not have the meal

S308

FIG. 7

**Blood glucose curve 1**

Blood glucose value
(mmol/L)

6.2

0    10    20    30    Time
(min)

**Blood glucose curve 2**

Blood glucose value
(mmol/L)

6.8

0    10    20    30    Time
(min)

FIG. 8(a)

EP 4 625 428 A1

**Blood glucose curve 1**

**Blood glucose curve 2**

Before time-domain transformation

After time-domain transformation

FIG. 8(b)

EP 4 625 428 A1

FIG. 8(c)

EP 4 625 428 A1

FIG. 8(d)

Legend:
— Transformed blood glucose curve 1
---- Transformed blood glucose curve 2
— Standard blood glucose curve

Axis labels: Blood glucose value (mmol/L); (6.5); Time (min); 0, 10, 20, (25), 30

Identify that a user is to start exercise — S401

Obtain exercise reference data of the user before the exercise — S402

Calculate an exercise risk score based on the exercise reference data — S403

Calculate an exercise risk threshold — S404

Output exercise prompt information based on the exercise risk score and the exercise risk threshold — S405

FIG. 9

Determine that a user starts exercise — S501

Obtain exercise reference data of the user during the exercise — S502

Calculate an exercise risk score based on the exercise reference data — S503

Determine whether the exercise risk score exceeds a threshold — S504

Output an exercise alarm — S505

FIG. 10

After exercise ends, obtain exercise reference data of a user in the single exercise ⟶ S601

Obtain exercise feedback information of the user ⟶ S602

Output an exercise evaluation report of the exercise ⟶ S603

FIG. 11

Identify an exogenous event by using an event identification model, or predict the exogenous event based on a work and rest rule of a user ⟶ S701

Obtain blood glucose values of the user within a period of time before a current time point ⟶ S702

Predict blood glucose values within a period of time after the current time point based on the exogenous event and the blood glucose values ⟶ S703

FIG. 12

Blood
glucose
value

Original blood glucose
– – – – curve when no exogenous
event is identified

——— Corrected blood glucose
curve after an exogenous
event is identified

Diet event+exercise event:
100 kcal carbohydrates+50
kcal running

Current time point          Time

FIG. 13

Blood
glucose
value

Original blood glucose
curve when no exogenous
– – – –        event is identified

——— Corrected blood glucose
curve after an exogenous
event is identified

Diet
event+medication
event:
200 kcal
carbohydrates+10 u
insulin

Current time point                              Time

FIG. 14

Electronic device 100

Antenna 1    Antenna 2

FIG. 15

| Application layer | Camera | Calendar | Maps | WLAN | Music | Messages |
|---|---|---|---|---|---|---|
| | Gallery | Phone | Navigation | Bluetooth | Videos | ... |

| Application framework layer | Window manager | Content provider | Phone manager | Resource manager |
|---|---|---|---|---|
| | Notification manager | View system | ... | |

| System library | Surface manager | Three-dimensional graphics processing library | Android runtime |
|---|---|---|---|
| | Two-dimensional graphics engine | Media library | ... | |

| Kernel layer | Display driver | Camera driver | |
|---|---|---|---|
| | Audio driver | Sensor driver | ... |

FIG. 16

Electronic device 200

Processor 201

Memory 202

Sensor 203

Temperature sensor 2031

Glucose detection sensor 2032

Wireless communication module 204

Antenna

FIG. 17

Electronic device 300

Processor 301

Memory 302

Display 304

Motor 305

Wireless communication module 306

Sensor 303

Gyroscope sensor 3031

Acceleration sensor 3032

Electrodermal activity sensor 3033

Touch sensor 3034

Bone conduction sensor 3035

Antenna

FIG. 18

## INTERNATIONAL SEARCH REPORT

| International application No. |
|---|
| **PCT/CN2023/143705** |

### A. CLASSIFICATION OF SUBJECT MATTER

G16H15/00(2018.01)i; A61B5/00(2006.01)i; G16H20/30(2018.01)i; G16H20/60(2018.01)i; G16H50/30(2018.01)i

According to International Patent Classification (IPC) or to both national classification and IPC

### B. FIELDS SEARCHED

Minimum documentation searched (classification system followed by classification symbols)

IPC: G16H A61B

Documentation searched other than minimum documentation to the extent that such documents are included in the fields searched

Electronic data base consulted during the international search (name of data base and, where practicable, search terms used)

CNABS; CNTXT; CNKI; WPABS; CJFD; VEN; USTXT; WOTXT; EPTXT: 血糖, 空腹, 进餐, 曲线, 指示, 显示, 运动, 预测, 模型, 训练, 评估, blood glucose, fasting, meals, curves, indication, display, exercise, prediction, model, training, assessment

### C. DOCUMENTS CONSIDERED TO BE RELEVANT

| Category* | Citation of document, with indication, where appropriate, of the relevant passages | Relevant to claim No. |
|---|---|---|
| X | CN 113017621 A (BESTECHNIC (SHANGHAI) CO., LTD.) 25 June 2021 (2021-06-25) description, paragraphs [0094]-[0105] | 1-7, 33-35 |
| X | CN 105160199 A (LIU YI) 16 December 2015 (2015-12-16) description, paragraphs [0028]-[0038], and figures 1-12 | 8-24, 33-35 |
| X | CN 112102953 A (PING AN TECHNOLOGY (SHENZHEN) CO., LTD.) 18 December 2020 (2020-12-18) description, paragraphs [0025]-[0051] | 25-35 |
| A | CN 108028076 A (MEDTRONIC MINIMED, INC.) 11 May 2018 (2018-05-11) entire document | 1-35 |
| A | CN 111329491 A (BOE TECHNOLOGY GROUP CO., LTD.) 26 June 2020 (2020-06-26) entire document | 1-35 |
| A | CN 112889114 A (MEDTRONIC MINIMED, INC.) 01 June 2021 (2021-06-01) entire document | 1-35 |
| A | US 2011047108 A1 (CHAKRABARTY NEILIN et al.) 24 February 2011 (2011-02-24) entire document | 1-35 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **11 March 2024** | **23 March 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/143705** |

| **C.** | **DOCUMENTS CONSIDERED TO BE RELEVANT** | |
| --- | --- | --- |
| Category* | Citation of document, with indication, where appropriate, of the relevant passages | Relevant to claim No. |
| A | US 2022202319 A1 (DEXCOM, INC.) 30 June 2022 (2022-06-30)<br>entire document | 1-35 |

Form PCT/ISA/210 (second sheet) (July 2022)

# EP 4 625 428 A1

## INTERNATIONAL SEARCH REPORT
### Information on patent family members

International application No.

**PCT/CN2023/143705**

| Patent document cited in search report | | | Publication date (day/month/year) | Patent family member(s) | | | Publication date (day/month/year) |
|---|---|---|---|---|---|---|---|
| CN | 113017621 | A | 25 June 2021 | CN | 113017621 | B | 21 November 2023 |
| CN | 105160199 | A | 16 December 2015 | None | | | |
| CN | 112102953 | A | 18 December 2020 | CN | 112102953 | B | 16 June 2023 |
| | | | | WO | 2022083124 | A1 | 28 April 2022 |
| CN | 108028076 | A | 11 May 2018 | US | 2020015739 | A1 | 16 January 2020 |
| | | | | WO | 2017035019 | A1 | 02 March 2017 |
| | | | | EP | 3338207 | A1 | 27 June 2018 |
| | | | | EP | 3338207 | B1 | 28 July 2021 |
| | | | | US | 2017049386 | A1 | 23 February 2017 |
| | | | | US | 10463297 | B2 | 05 November 2019 |
| | | | | US | 2022181003 | A1 | 09 June 2022 |
| | | | | CA | 2994723 | A1 | 02 March 2017 |
| | | | | CN | 108028076 | B | 24 March 2023 |
| CN | 111329491 | A | 26 June 2020 | None | | | |
| CN | 112889114 | A | 01 June 2021 | WO | 2020092573 | A1 | 07 May 2020 |
| | | | | CA | 3117825 | A1 | 07 May 2020 |
| | | | | US | 2020135320 | A1 | 30 April 2020 |
| | | | | US | 2022301679 | A1 | 22 September 2022 |
| | | | | EP | 3874516 | A1 | 08 September 2021 |
| | | | | US | 2020135319 | A1 | 30 April 2020 |
| | | | | US | 11367516 | B2 | 21 June 2022 |
| | | | | AU | 2019370301 | A1 | 03 June 2021 |
| | | | | JP | 2022506115 | A | 17 January 2022 |
| | | | | KR | 20210086618 | A | 08 July 2021 |
| US | 2011047108 | A1 | 24 February 2011 | CA | 2708243 | A1 | 21 February 2011 |
| | | | | US | 8589082 | B2 | 19 November 2013 |
| US | 2022202319 | A1 | 30 June 2022 | CA | 3193444 | A1 | 07 July 2022 |
| | | | | AU | 2021411975 | A1 | 27 July 2023 |
| | | | | WO | 2022147316 | A1 | 07 July 2022 |

Form PCT/ISA/210 (patent family annex) (July 2022)

**REFERENCES CITED IN THE DESCRIPTION**

**Patent documents cited in the description**

- CN 202310003669
- CN 202310258733